# EUROPEAN PATENT APPLICATION

(11) **EP 1 803 807 A2**
(43) Date of publication of application: **04.07.2007**
(21) Application number: 07000976.6
(22) Date of filing: 18.05.2001
(51) Int. Cl.: C12N 1/36, C12N 15/31, C07K 14/22, C07K 16/12, A61K 39/095, A61P 37/02

(54) **Virulence genes, proteins, and their use**

(30) Priority: 18.05.2000 GB 0012079
(62) Divisional of application: 01931888.0
(71) Applicant: Microscience Limited, Wokingham, Berkshire RG41 5TU (GB)
(72) Inventor: Tang, Christopher, Imperial College of Science, Armstrong Road, London SW7 2AZ (GB)
(74) Representative: Jappy, John William Graham

(57) **Abstract**

A series of genes form *Neisseria meningitidis* are shown to encode products which are responsible for DNA uptake. The identification of these genes therefore allows attenuated microorganisms to be produced that have a reduced ability to take up DNA. Microorganisms of the invention may be used in the production of genetically stable mutant microorganisms. The genes or their encoded products can be used in the manufacture of vaccines for therapeutic application.

SEQUENCE LISTING

<110> Microscience Limited

<120> Virulence Genes, Proteins, and Their Use

<130> REP06443WO

<140> (not yet known)
<141> 2001-05-18

<150> 0012079.0
<151> 2000-05-18

<160> 22

<170> PatentIn Ver. 2.1

<210> 1
<211> 2040
<212> DNA
<213> Neisseria meningitidis

<220>
<221> CDS
<222> (1)..(2040)

<400> 1
atg atg tcg ccc gaa acc cga aaa cag ctc aaa atc acc gat gtt tcc 48
Met Met Ser Pro Glu Thr Arg Lys Gln Leu Lys Ile Thr Asp Val Ser
1               5                   10                  15

gcc aag aag ctc gac aaa ctc aac ctc cat acc gcg tgg gat ttg gtg 96
Ala Lys Lys Leu Asp Lys Leu Asn Leu His Thr Ala Trp Asp Leu Val
            20                  25                  30

ttg cac ctg ccg ctg cgt tac gag gac gag acg cac att atg ccg att 144
Leu His Leu Pro Leu Arg Tyr Glu Asp Glu Thr His Ile Met Pro Ile
        35                  40                  45

aag gac gcg ccg att ggc gtg ccg tgt cag gtc gag ggg gag gtt atc 192
Lys Asp Ala Pro Ile Gly Val Pro Cys Gln Val Glu Gly Glu Val Ile
    50                  55                  60

cat cag gaa gta acg ttc aaa ccg cgc aag cag ctg att gtc caa atc 240
His Gln Glu Val Thr Phe Lys Pro Arg Lys Gln Leu Ile Val Gln Ile
65                  70                  75                  80

gcc gac ggc tcc ggc agc gtc ctt ttt ctg cgc ttc atc cac ttt tac     288
Ala Asp Gly Ser Gly Ser Val Leu Phe Leu Arg Phe Ile His Phe Tyr
                85                  90                  95

gcc agc cat cag aaa cag acg gcg atc ggc aaa cgc atc cgc gcc gtg     336
Ala Ser His Gln Lys Gln Thr Ala Ile Gly Lys Arg Ile Arg Ala Val
            100                 105                 110

ggc gaa atc aaa cac ggc ttt tac ggc gac gag atg att cat ccc aaa     384
Gly Glu Ile Lys His Gly Phe Tyr Gly Asp Glu Met Ile His Pro Lys
        115                 120                 125

atc cgc gat gcc gag ggc ggc ggt ttg gcg gaa agc ctc acg ccg gtt     432
Ile Arg Asp Ala Glu Gly Gly Gly Leu Ala Glu Ser Leu Thr Pro Val
    130                 135                 140

tac ccg acc gta aac ggt ttg aac cag ccc act ttg cgc cgt att att     480
Tyr Pro Thr Val Asn Gly Leu Asn Gln Pro Thr Leu Arg Arg Ile Ile
145                 150                 155                 160

cag acg gcg ttg gac gtt acg ccg ctg cac gac acg ctg ccc gat gcc     528
Gln Thr Ala Leu Asp Val Thr Pro Leu His Asp Thr Leu Pro Asp Ala
                165                 170                 175

ctg ctg tgc cgt ctg aag ctg cca cac ctc gcc gaa agc ctg cgt ctt     576
Leu Leu Cys Arg Leu Lys Leu Pro His Leu Ala Glu Ser Leu Arg Leu
            180                 185                 190

ttg cat tcg ccg ccg ccg agt ttc acg att cat cag ctt tca gac ggc     624
Leu His Ser Pro Pro Pro Ser Phe Thr Ile His Gln Leu Ser Asp Gly
        195                 200                 205

acg ctg cct gca tgg caa cgg ctc aaa ttc gac gaa ctt ttg gcg caa     672
Thr Leu Pro Ala Trp Gln Arg Leu Lys Phe Asp Glu Leu Leu Ala Gln
    210                 215                 220

cag ctt tcg atg cgc ttg gcg cga cag aag cgt atc ggc ggc acg gcg     720
Gln Leu Ser Met Arg Leu Ala Arg Gln Lys Arg Ile Gly Gly Thr Ala
225                 230                 235                 240

gcg gca ttg ggc ggc gac ggc aca ttg acc caa gcc ctg cgc caa gcc     768
Ala Ala Leu Gly Gly Asp Gly Thr Leu Thr Gln Ala Leu Arg Gln Ala
                245                 250                 255

ctg ccg ttt gcc ctg acc gat gcg caa gaa aaa gtt gtt tcc gaa atc     816
Leu Pro Phe Ala Leu Thr Asp Ala Gln Glu Lys Val Val Ser Glu Ile
            260                 265                 270

tgc cgc gat atg gcg caa acc tac ccg atg cac cgc ctg ctg cag ggc 864
Cys Arg Asp Met Ala Gln Thr Tyr Pro Met His Arg Leu Leu Gln Gly
        275                 280                 285

gat gtc ggc agc ggc aaa acc att gtg gct gct ttg tct gct ttg acg 912
Asp Val Gly Ser Gly Lys Thr Ile Val Ala Ala Leu Ser Ala Leu Thr
    290                 295                 300

gct atc gaa tcc ggt gcg cag gtg gct gta atg gcg ccc act gaa atc 960
Ala Ile Glu Ser Gly Ala Gln Val Ala Val Met Ala Pro Thr Glu Ile
305                 310                 315                 320

ctt gcc gaa cag cat ttt att aaa ttt aaa caa tgg ctc gaa cct ttg 1008
Leu Ala Glu Gln His Phe Ile Lys Phe Lys Gln Trp Leu Glu Pro Leu
                325                 330                 335

ggc att gaa gtt gtc cgc ctt ttt ggc agt ttg cgt aaa aaa gcc aaa 1056
Gly Ile Glu Val Val Arg Leu Phe Gly Ser Leu Arg Lys Lys Ala Lys
            340                 345                 350

gac gaa gcc aaa gcc aaa ctg gct gac ggc agc gtc aaa atc gcc gtc 1104
Asp Glu Ala Lys Ala Lys Leu Ala Asp Gly Ser Val Lys Ile Ala Val
        355                 360                 365

ggc acg cac gcc ctg ttt tca gac ggc gtg gcg ttt cac aat ttg ggc 1152
Gly Thr His Ala Leu Phe Ser Asp Gly Val Ala Phe His Asn Leu Gly
    370                 375                 380

ttg acc att gtg gac gaa cag cac cgt ttc ggc gtt gcc caa cgc ctc 1200
Leu Thr Ile Val Asp Glu Gln His Arg Phe Gly Val Ala Gln Arg Leu
385                 390                 395                 400

gcc ctc aaa aac aaa ggg cgc gaa gtc cat cag ctg atg atg tcc gcc 1248
Ala Leu Lys Asn Lys Gly Arg Glu Val His Gln Leu Met Met Ser Ala
                405                 410                 415

acg ccc atc ccg cgc acg ctt gcg atg agt ttt ttc gcc gat ttg gac 1296
Thr Pro Ile Pro Arg Thr Leu Ala Met Ser Phe Phe Ala Asp Leu Asp
            420                 425                 430

gtg tcc gtc atc gac gaa ttg ccg ccc ggg cgc aca ccg att aaa acg 1344
Val Ser Val Ile Asp Glu Leu Pro Pro Gly Arg Thr Pro Ile Lys Thr
        435                 440                 445

cgc ctc gtc aac aac gtc cgc cgc gcc gaa gtc gaa ggc ttc gtc ctc 1392
Arg Leu Val Asn Asn Val Arg Arg Ala Glu Val Glu Gly Phe Val Leu
    450                 455                 460

ggc act tgc cga aaa ggg cgg cag gcg tat tgg gtc tgc cca ttg att 1440
Gly Thr Cys Arg Lys Gly Arg Gln Ala Tyr Trp Val Cys Pro Leu Ile
465                 470                 475                 480

gaa gaa agc gaa acc ctg caa ctg caa acc gcc gcc gaa acc ctc gcc 1488
Glu Glu Ser Glu Thr Leu Gln Leu Gln Thr Ala Ala Glu Thr Leu Ala
                485                 490                 495

cgg ctt cag acg gca ttg ccc gaa ctc aat atc gga ctg gta cac ggg 1536
Arg Leu Gln Thr Ala Leu Pro Glu Leu Asn Ile Gly Leu Val His Gly
            500                 505                 510

cgc atg aag gcc gcc gaa aag gcc gaa gtg atg gcg cgg ttt tct tcg 1584
Arg Met Lys Ala Ala Glu Lys Ala Glu Val Met Ala Arg Phe Ser Ser
        515                 520                 525

ggg ggg ctg aac gtc ttg gtc gcc acc acc gtc atc gaa gtc ggc gta 1632
Gly Gly Leu Asn Val Leu Val Ala Thr Thr Val Ile Glu Val Gly Val
    530                 535                 540

gat gtg ccc aat gcc gcc ctg atg gtc atc gaa cac gcc gag cgc atg 1680
Asp Val Pro Asn Ala Ala Leu Met Val Ile Glu His Ala Glu Arg Met
545                 550                 555                 560

ggc ttg gcg cag ctt cac caa tta cgc gga cgg gta ggg cgc ggc gcg 1728
Gly Leu Ala Gln Leu His Gln Leu Arg Gly Arg Val Gly Arg Gly Ala
                565                 570                 575

gcg gaa agc gtg tgc gtc ctc ctg ttt gcc gaa ccc ttg ggc gaa ctc 1776
Ala Glu Ser Val Cys Val Leu Leu Phe Ala Glu Pro Leu Gly Glu Leu
            580                 585                 590

gcc aaa gcg cgg ctg aaa gtc atc tac gaa cac acc gac ggc ttc gaa 1824
Ala Lys Ala Arg Leu Lys Val Ile Tyr Glu His Thr Asp Gly Phe Glu
        595                 600                 605

atc gcc cgc caa gac ctc aat atc cgc ggc ccg ggc gag ttt ctc ggc 1872
Ile Ala Arg Gln Asp Leu Asn Ile Arg Gly Pro Gly Glu Phe Leu Gly
    610                 615                 620

gcg cgc caa agc ggc gtg cct atg ctg cgt ttc gcc aaa ctc gaa gaa 1920
Ala Arg Gln Ser Gly Val Pro Met Leu Arg Phe Ala Lys Leu Glu Glu
625                 630                 635                 640

gac ctg cac ctt ttg gaa cag gcg cgc gaa acc gcc ccg atg ctg att 1968
Asp Leu His Leu Leu Glu Gln Ala Arg Glu Thr Ala Pro Met Leu Ile
                645                 650                 655

gag caa aac ccc gaa atc gtc gaa gcg cat ttg gca agg tgg ctt tcc 2016
Glu Gln Asn Pro Glu Ile Val Glu Ala His Leu Ala Arg Trp Leu Ser
            660                 665                 670

ggc agg gaa ggg tat ttg ggc gtg 2040
Gly Arg Glu Gly Tyr Leu Gly Val
        675                 680

<210> 2
<211> 680
<212> PRT
<213> Neisseria meningitidis

<400> 2
Met Met Ser Pro Glu Thr Arg Lys Gln Leu Lys Ile Thr Asp Val Ser
1               5                   10                  15

Ala Lys Lys Leu Asp Lys Leu Asn Leu His Thr Ala Trp Asp Leu Val
            20                  25                  30

Leu His Leu Pro Leu Arg Tyr Glu Asp Glu Thr His Ile Met Pro Ile
        35                  40                  45

Lys Asp Ala Pro Ile Gly Val Pro Cys Gln Val Glu Gly Glu Val Ile
    50                  55                  60

His Gln Glu Val Thr Phe Lys Pro Arg Lys Gln Leu Ile Val Gln Ile
65                  70                  75                  80

Ala Asp Gly Ser Gly Ser Val Leu Phe Leu Arg Phe Ile His Phe Tyr
                85                  90                  95

Ala Ser His Gln Lys Gln Thr Ala Ile Gly Lys Arg Ile Arg Ala Val
            100                 105                 110

Gly Glu Ile Lys His Gly Phe Tyr Gly Asp Glu Met Ile His Pro Lys
        115                 120                 125

Ile Arg Asp Ala Glu Gly Gly Gly Leu Ala Glu Ser Leu Thr Pro Val
    130                 135                 140

Tyr Pro Thr Val Asn Gly Leu Asn Gln Pro Thr Leu Arg Arg Ile Ile
145                 150                 155                 160

Gln Thr Ala Leu Asp Val Thr Pro Leu His Asp Thr Leu Pro Asp Ala
                165                 170                 175

Leu Leu Cys Arg Leu Lys Leu Pro His Leu Ala Glu Ser Leu Arg Leu
            180                 185                 190

Leu His Ser Pro Pro Pro Ser Phe Thr Ile His Gln Leu Ser Asp Gly
        195                 200                 205

Thr Leu Pro Ala Trp Gln Arg Leu Lys Phe Asp Glu Leu Leu Ala Gln
    210                 215                 220

Gln Leu Ser Met Arg Leu Ala Arg Gln Lys Arg Ile Gly Gly Thr Ala
225                 230                 235                 240

Ala Ala Leu Gly Gly Asp Gly Thr Leu Thr Gln Ala Leu Arg Gln Ala
                245                 250                 255

Leu Pro Phe Ala Leu Thr Asp Ala Gln Glu Lys Val Val Ser Glu Ile
            260                 265                 270

Cys Arg Asp Met Ala Gln Thr Tyr Pro Met His Arg Leu Leu Gln Gly
        275                 280                 285

Asp Val Gly Ser Gly Lys Thr Ile Val Ala Ala Leu Ser Ala Leu Thr
    290                 295                 300

Ala Ile Glu Ser Gly Ala Gln Val Ala Val Met Ala Pro Thr Glu Ile
305                 310                 315                 320

Leu Ala Glu Gln His Phe Ile Lys Phe Lys Gln Trp Leu Glu Pro Leu
                325                 330                 335

Gly Ile Glu Val Val Arg Leu Phe Gly Ser Leu Arg Lys Lys Ala Lys
            340                 345                 350

Asp Glu Ala Lys Ala Lys Leu Ala Asp Gly Ser Val Lys Ile Ala Val
        355                 360                 365

Gly Thr His Ala Leu Phe Ser Asp Gly Val Ala Phe His Asn Leu Gly
    370                 375                 380

Leu Thr Ile Val Asp Glu Gln His Arg Phe Gly Val Ala Gln Arg Leu
385                 390                 395                 400

Ala Leu Lys Asn Lys Gly Arg Glu Val His Gln Leu Met Met Ser Ala
                405                 410                 415

Thr Pro Ile Pro Arg Thr Leu Ala Met Ser Phe Phe Ala Asp Leu Asp
            420                 425                 430

Val Ser Val Ile Asp Glu Leu Pro Pro Gly Arg Thr Pro Ile Lys Thr
        435                 440                 445

Arg Leu Val Asn Asn Val Arg Arg Ala Glu Val Glu Gly Phe Val Leu
    450                 455                 460

Gly Thr Cys Arg Lys Gly Arg Gln Ala Tyr Trp Val Cys Pro Leu Ile
465                 470                 475                 480

Glu Glu Ser Glu Thr Leu Gln Leu Gln Thr Ala Ala Glu Thr Leu Ala
                485                 490                 495

Arg Leu Gln Thr Ala Leu Pro Glu Leu Asn Ile Gly Leu Val His Gly
            500                 505                 510

Arg Met Lys Ala Ala Glu Lys Ala Glu Val Met Ala Arg Phe Ser Ser
        515                 520                 525

Gly Gly Leu Asn Val Leu Val Ala Thr Thr Val Ile Glu Val Gly Val
    530                 535                 540

Asp Val Pro Asn Ala Ala Leu Met Val Ile Glu His Ala Glu Arg Met
545                 550                 555                 560

Gly Leu Ala Gln Leu His Gln Leu Arg Gly Arg Val Gly Arg Gly Ala
                565                 570                 575

Ala Glu Ser Val Cys Val Leu Leu Phe Ala Glu Pro Leu Gly Glu Leu
            580                 585                 590

Ala Lys Ala Arg Leu Lys Val Ile Tyr Glu His Thr Asp Gly Phe Glu
        595                 600                 605

Ile Ala Arg Gln Asp Leu Asn Ile Arg Gly Pro Gly Glu Phe Leu Gly
    610                 615                 620

Ala Arg Gln Ser Gly Val Pro Met Leu Arg Phe Ala Lys Leu Glu Glu
625                 630                 635                 640

Asp Leu His Leu Leu Glu Gln Ala Arg Glu Thr Ala Pro Met Leu Ile
                645                 650                 655

Glu Gln Asn Pro Glu Ile Val Glu Ala His Leu Ala Arg Trp Leu Ser
            660                 665                 670

Gly Arg Glu Gly Tyr Leu Gly Val
        675                 680

<210> 3
<211> 1257
<212> DNA
<213> Neisseria meningitidis

<220>
<221> CDS
<222> (1)..(1257)

<400> 3
atg cac gtc tcc gaa tta caa acc ctg cac att tcc aaa ctc tta gaa 48
Met His Val Ser Glu Leu Gln Thr Leu His Ile Ser Lys Leu Leu Glu
1               5                   10                  15

ttg gcg gaa gaa cac ggc atc gaa aac gcc aac cga ttc cgc aaa caa 96
Leu Ala Glu Glu His Gly Ile Glu Asn Ala Asn Arg Phe Arg Lys Gln
            20                  25                  30

gat ctc gta ttt gcc atc gtc cgc cag atg atg aaa aaa ggc gag ggt 144
Asp Leu Val Phe Ala Ile Val Arg Gln Met Met Lys Lys Gly Glu Gly
        35                  40                  45

ttc acc tgc tcc ggc acg ctt gaa atc ctg ccc gac ggc ttc ggc ttc 192
Phe Thr Cys Ser Gly Thr Leu Glu Ile Leu Pro Asp Gly Phe Gly Phe
    50                  55                  60

ctc cgc agc gcg gac acg tcc tat ctt gcc ggc ccc gac gac atc tat 240
Leu Arg Ser Ala Asp Thr Ser Tyr Leu Ala Gly Pro Asp Asp Ile Tyr
65                  70                  75                  80

gtc tcg ccc acc caa atc cgc cgc ttc aac ctg cat acg ggc gac acc 288
Val Ser Pro Thr Gln Ile Arg Arg Phe Asn Leu His Thr Gly Asp Thr
                85                  90                  95

atc gaa gga agc gtg cgc gtc cca aaa gac aac gaa cgc tat ttt gcc 336
Ile Glu Gly Ser Val Arg Val Pro Lys Asp Asn Glu Arg Tyr Phe Ala
            100                 105                 110

ctg gtc agg ctt gat acc atc aac ggc gac cac ccg gaa gta tgc cgc 384
Leu Val Arg Leu Asp Thr Ile Asn Gly Asp His Pro Glu Val Cys Arg
        115                 120                 125

cat aaa atc ctg ttt gaa aac ctg acc ccg ctg ttt ccg acc gaa cag 432
His Lys Ile Leu Phe Glu Asn Leu Thr Pro Leu Phe Pro Thr Glu Gln
    130                 135                 140

ttg aag ctg gaa cgc gac tta aag tcc gaa gaa aac ctg acc gga cgt    480
Leu Lys Leu Glu Arg Asp Leu Lys Ser Glu Glu Asn Leu Thr Gly Arg
145                 150                 155                 160

gcc atc gac ctg att tcc cct atc ggc aaa ggt cag cgc gcc ctc ttg    528
Ala Ile Asp Leu Ile Ser Pro Ile Gly Lys Gly Gln Arg Ala Leu Leu
                165                 170                 175

gtt gcc ccg ccc aaa agc ggt aaa acc gtg atg ctg caa aac att gcc    576
Val Ala Pro Pro Lys Ser Gly Lys Thr Val Met Leu Gln Asn Ile Ala
            180                 185                 190

cac gcc gtt acc tca aac tat ccc gaa gtc gaa ctc atc gtc ctc ttg    624
His Ala Val Thr Ser Asn Tyr Pro Glu Val Glu Leu Ile Val Leu Leu
        195                 200                 205

att gac gag cgt ccc gaa gaa gta acc gaa atg agc cgt tcc gtc cgc    672
Ile Asp Glu Arg Pro Glu Glu Val Thr Glu Met Ser Arg Ser Val Arg
    210                 215                 220

ggc gaa gtg gtt tcc tcc acc ttt gac gaa ccg gcg caa cgc cac gtc    720
Gly Glu Val Val Ser Ser Thr Phe Asp Glu Pro Ala Gln Arg His Val
225                 230                 235                 240

caa gtt gcc gaa atg gtg ctt gaa aaa gcc aag cgt atg gta gaa cac    768
Gln Val Ala Glu Met Val Leu Glu Lys Ala Lys Arg Met Val Glu His
                245                 250                 255

aaa aaa gac gtg gtc atc ctg ctg gat tcg att acc cgc ctt gcc cgc    816
Lys Lys Asp Val Val Ile Leu Leu Asp Ser Ile Thr Arg Leu Ala Arg
            260                 265                 270

gcc tac aat acc gtc gtg cct acc tcg ggc aaa atc cta acc ggc ggt    864
Ala Tyr Asn Thr Val Val Pro Thr Ser Gly Lys Ile Leu Thr Gly Gly
        275                 280                 285

gtc gat gcc aac gca ctg cac cgc ccc aaa cgt ttc ttc ggc gcg gcg    912
Val Asp Ala Asn Ala Leu His Arg Pro Lys Arg Phe Phe Gly Ala Ala
    290                 295                 300

cgc aac gtg gaa gaa ggc ggt tcg ctg acc atc atc gcc acc gca ttg    960
Arg Asn Val Glu Glu Gly Gly Ser Leu Thr Ile Ile Ala Thr Ala Leu
305                 310                 315                 320

gtt gaa acc ggc agc cgt atg gac gat gtg att tac gaa gaa ttc aaa    1008
Val Glu Thr Gly Ser Arg Met Asp Asp Val Ile Tyr Glu Glu Phe Lys
                325                 330                 335

ggc acc ggc aat atg gaa ttg cac ctt gac cgc cgt atg gcg gaa aaa 1056
Gly Thr Gly Asn Met Glu Leu His Leu Asp Arg Arg Met Ala Glu Lys
            340                 345                 350

cgc ctc ttc ccc gcc atc aac atc aac aaa tcc ggc acg cgc cgc gaa 1104
Arg Leu Phe Pro Ala Ile Asn Ile Asn Lys Ser Gly Thr Arg Arg Glu
        355                 360                 365

gag ctg ctt gtg ccg aac gac cag ttg caa cgt atg tgg ctc tta cgc 1152
Glu Leu Leu Val Pro Asn Asp Gln Leu Gln Arg Met Trp Leu Leu Arg
    370                 375                 380

aag ttc ctg cac ccg atg gac gaa atc gag gca gcc gaa ttt tta atc 1200
Lys Phe Leu His Pro Met Asp Glu Ile Glu Ala Ala Glu Phe Leu Ile
385                 390                 395                 400

ggg aaa atc aaa gcc tct aaa aac aat gat gat ttc ttt gaa ctg atg 1248
Gly Lys Ile Lys Ala Ser Lys Asn Asn Asp Asp Phe Phe Glu Leu Met
                405                 410                 415

cgc ggc aaa 1257
Arg Gly Lys

<210> 4
<211> 419
<212> PRT
<213> Neisseria meningitidis

<400> 4
Met His Val Ser Glu Leu Gln Thr Leu His Ile Ser Lys Leu Leu Glu
1               5                   10                  15

Leu Ala Glu Glu His Gly Ile Glu Asn Ala Asn Arg Phe Arg Lys Gln
            20                  25                  30

Asp Leu Val Phe Ala Ile Val Arg Gln Met Met Lys Lys Gly Glu Gly
        35                  40                  45

Phe Thr Cys Ser Gly Thr Leu Glu Ile Leu Pro Asp Gly Phe Gly Phe
    50                  55                  60

Leu Arg Ser Ala Asp Thr Ser Tyr Leu Ala Gly Pro Asp Asp Ile Tyr
65                  70                  75                  80

Val Ser Pro Thr Gln Ile Arg Arg Phe Asn Leu His Thr Gly Asp Thr
                85                  90                  95

Ile Glu Gly Ser Val Arg Val Pro Lys Asp Asn Glu Arg Tyr Phe Ala
            100                 105                 110

Leu Val Arg Leu Asp Thr Ile Asn Gly Asp His Pro Glu Val Cys Arg
        115                 120                 125

His Lys Ile Leu Phe Glu Asn Leu Thr Pro Leu Phe Pro Thr Glu Gln
    130                 135                 140

Leu Lys Leu Glu Arg Asp Leu Lys Ser Glu Glu Asn Leu Thr Gly Arg
145                 150                 155                 160

Ala Ile Asp Leu Ile Ser Pro Ile Gly Lys Gly Gln Arg Ala Leu Leu
                165                 170                 175

Val Ala Pro Pro Lys Ser Gly Lys Thr Val Met Leu Gln Asn Ile Ala
            180                 185                 190

His Ala Val Thr Ser Asn Tyr Pro Glu Val Glu Leu Ile Val Leu Leu
        195                 200                 205

Ile Asp Glu Arg Pro Glu Glu Val Thr Glu Met Ser Arg Ser Val Arg
    210                 215                 220

Gly Glu Val Val Ser Ser Thr Phe Asp Glu Pro Ala Gln Arg His Val
225                 230                 235                 240

Gln Val Ala Glu Met Val Leu Glu Lys Ala Lys Arg Met Val Glu His
                245                 250                 255

Lys Lys Asp Val Val Ile Leu Leu Asp Ser Ile Thr Arg Leu Ala Arg
            260                 265                 270

Ala Tyr Asn Thr Val Val Pro Thr Ser Gly Lys Ile Leu Thr Gly Gly
        275                 280                 285

Val Asp Ala Asn Ala Leu His Arg Pro Lys Arg Phe Phe Gly Ala Ala
    290                 295                 300

Arg Asn Val Glu Glu Gly Gly Ser Leu Thr Ile Ile Ala Thr Ala Leu
305                 310                 315                 320

Val Glu Thr Gly Ser Arg Met Asp Asp Val Ile Tyr Glu Glu Phe Lys
                325                 330                 335

Gly Thr Gly Asn Met Glu Leu His Leu Asp Arg Arg Met Ala Glu Lys
            340                 345                 350

Arg Leu Phe Pro Ala Ile Asn Ile Asn Lys Ser Gly Thr Arg Arg Glu
        355                 360                 365

Glu Leu Leu Val Pro Asn Asp Gln Leu Gln Arg Met Trp Leu Leu Arg
    370                 375                 380

Lys Phe Leu His Pro Met Asp Glu Ile Glu Ala Ala Glu Phe Leu Ile
385                 390                 395                 400

Gly Lys Ile Lys Ala Ser Lys Asn Asn Asp Asp Phe Phe Glu Leu Met
                405                 410                 415

Arg Gly Lys

<210> 5
<211> 599
<212> DNA
<213> Neisseria meningitidis

<220>
<221> CDS
<222> (1)..(597)

<400> 5
atg aac aat tta atc aaa atc aac ctc ctc ccc tac agg gaa gag atg    48
Met Asn Asn Leu Ile Lys Ile Asn Leu Leu Pro Tyr Arg Glu Glu Met
  1               5                  10                  15

aac aag cgc aaa cag cag cag ttt aaa acg ctg atg tac ggt gcc gtg    96
Asn Lys Arg Lys Gln Gln Gln Phe Lys Thr Leu Met Tyr Gly Ala Val
             20                  25                  30

ctg acg ggc gtt gcc gcc gtt gcg gca acc tac ctg ttt atc gac aat   144
Leu Thr Gly Val Ala Ala Val Ala Ala Thr Tyr Leu Phe Ile Asp Asn
         35                  40                  45

atg atc aat aac cag tcg gaa aga aac acg ctg ctg gaa acc tcc atc   192
Met Ile Asn Asn Gln Ser Glu Arg Asn Thr Leu Leu Glu Thr Ser Ile
     50                  55                  60

gca cac ttg gat acc gag ctg tcg gaa ata caa aag ctc aaa cag gaa   240
Ala His Leu Asp Thr Glu Leu Ser Glu Ile Gln Lys Leu Lys Gln Glu
 65                  70                  75                  80

aaa gat gcc ttc ctg att aag aaa aac aaa atc gag gag ctc cag ctc   288

Lys Asp Ala Phe Leu Ile Lys Lys Asn Lys Ile Glu Glu Leu Gln Leu
                85                  90                  95

aaa cgc ctc caa gcc gca aaa atc ctc gac agc ctg aat gag gcc gtc 336
Lys Arg Leu Gln Ala Ala Lys Ile Leu Asp Ser Leu Asn Glu Ala Val
            100                 105                 110

ccc gga agc acc tac ctg acc tcg ctg gat gcc gtt acc gcc gac tct 384
Pro Gly Ser Thr Tyr Leu Thr Ser Leu Asp Ala Val Thr Ala Asp Ser
        115                 120                 125

tat cgg ctc agc ggc agg aca tcc agc gac aac cgc gtt gcc gcc atg 432
Tyr Arg Leu Ser Gly Arg Thr Ser Ser Asp Asn Arg Val Ala Ala Met
    130                 135                 140

atg agg gcg atg ccc aat acc ggc ata ttc aag caa ccc gaa ttg tta 480
Met Arg Ala Met Pro Asn Thr Gly Ile Phe Lys Gln Pro Glu Leu Leu
145                 150                 155                 160

agc atc aag aaa aac aat tcg cat caa gaa ttt acc ctt cag gca aca 528
Ser Ile Lys Lys Asn Asn Ser His Gln Glu Phe Thr Leu Gln Ala Thr
                165                 170                 175

tta caa ccc atc gta aag gcg gcc gaa tcc aaa gag aat ccg gct tcg 576
Leu Gln Pro Ile Val Lys Ala Ala Glu Ser Lys Glu Asn Pro Ala Ser
            180                 185                 190

gga aac gca cag gag gca aac tg 599
Gly Asn Ala Gln Glu Ala Asn
        195

<210> 6
<211> 199
<212> PRT
<213> Neisseria meningitidis

<400> 6
Met Asn Asn Leu Ile Lys Ile Asn Leu Leu Pro Tyr Arg Glu Glu Met
1               5                   10                  15

Asn Lys Arg Lys Gln Gln Gln Phe Lys Thr Leu Met Tyr Gly Ala Val
            20                  25                  30

Leu Thr Gly Val Ala Ala Val Ala Ala Thr Tyr Leu Phe Ile Asp Asn
        35                  40                  45

Met Ile Asn Asn Gln Ser Glu Arg Asn Thr Leu Leu Glu Thr Ser Ile

    50                  55                  60

Ala His Leu Asp Thr Glu Leu Ser Glu Ile Gln Lys Leu Lys Gln Glu
65                  70                  75                  80

Lys Asp Ala Phe Leu Ile Lys Lys Asn Lys Ile Glu Glu Leu Gln Leu
                85                  90                  95

Lys Arg Leu Gln Ala Ala Lys Ile Leu Asp Ser Leu Asn Glu Ala Val
            100                 105                 110

Pro Gly Ser Thr Tyr Leu Thr Ser Leu Asp Ala Val Thr Ala Asp Ser
        115                 120                 125

Tyr Arg Leu Ser Gly Arg Thr Ser Ser Asp Asn Arg Val Ala Ala Met
    130                 135                 140

Met Arg Ala Met Pro Asn Thr Gly Ile Phe Lys Gln Pro Glu Leu Leu
145                 150                 155                 160

Ser Ile Lys Lys Asn Asn Ser His Gln Glu Phe Thr Leu Gln Ala Thr
                165                 170                 175

Leu Gln Pro Ile Val Lys Ala Ala Glu Ser Lys Glu Asn Pro Ala Ser
            180                 185                 190

Gly Asn Ala Gln Glu Ala Asn
        195

<210> 7
<211> 1773
<212> DNA
<213> Neisseria meningitidis

<220>
<221> CDS
<222> (1)..(1773)

<400> 7
atg agt atc gtg ctg cac ggc gtg gcg gcg ggc aaa ggc att gcc gtc 48
Met Ser Ile Val Leu His Gly Val Ala Ala Gly Lys Gly Ile Ala Val
1               5                   10                  15

ggt tgc gcc cac ctg att gcg cgc ggt acg gag gaa gtg ccg cag tat 96
Gly Cys Ala His Leu Ile Ala Arg Gly Thr Glu Glu Val Pro Gln Tyr
            20                  25                  30

gat gtt gcg gag gcg gac acc gat gcc gaa gcc gaa cgt ttc gat gcc 144
Asp Val Ala Glu Ala Asp Thr Asp Ala Glu Ala Glu Arg Phe Asp Ala
        35                  40                  45

gcc gtc aaa gcc acg cgc aaa gag ttg gaa cag ctc cgc agc gcg att 192
Ala Val Lys Ala Thr Arg Lys Glu Leu Glu Gln Leu Arg Ser Ala Ile
    50                  55                  60

ccc gaa aac gcc ccg acc gag ttg ggc gcg ttc atc tcg ctg cac ctg 240
Pro Glu Asn Ala Pro Thr Glu Leu Gly Ala Phe Ile Ser Leu His Leu
65                  70                  75                  80

atg ctc ttg acc gat gtt acc ttg tcg cgc gaa ccc gtc gat att tta 288
Met Leu Leu Thr Asp Val Thr Leu Ser Arg Glu Pro Val Asp Ile Leu
                85                  90                  95

agg gaa caa aaa atc aac gcc gag tgg gca ttg aag cag cag agc gac 336
Arg Glu Gln Lys Ile Asn Ala Glu Trp Ala Leu Lys Gln Gln Ser Asp
            100                 105                 110

aaa ctc gcc gcc caa ttc gac aat atg gac gat gcc tat ttg cgc gaa 384
Lys Leu Ala Ala Gln Phe Asp Asn Met Asp Asp Ala Tyr Leu Arg Glu
        115                 120                 125

cgc aag cag gat atg ctg caa gtc gtc cgc cgc atc cac aac aac ctg 432
Arg Lys Gln Asp Met Leu Gln Val Val Arg Arg Ile His Asn Asn Leu
    130                 135                 140

atc ggg cag ggc aac gag ttg gaa gtt gcc gac aac ctg ttt gac gaa 480
Ile Gly Gln Gly Asn Glu Leu Glu Val Ala Asp Asn Leu Phe Asp Glu
145                 150                 155                 160

acc gtt ctg att gca aac gac ctt tcg ccc gcc gac acg gtt ttg ttt 528
Thr Val Leu Ile Ala Asn Asp Leu Ser Pro Ala Asp Thr Val Leu Phe
                165                 170                 175

aaa gag cag cgc att gcc gcc ttc gtt acc gat gcc ggc ggc ccc acc 576
Lys Glu Gln Arg Ile Ala Ala Phe Val Thr Asp Ala Gly Gly Pro Thr
            180                 185                 190

ggg cat acg gcg att ttg ggc agg agc ttg gac atc ccg tcc gtc gtc 624
Gly His Thr Ala Ile Leu Gly Arg Ser Leu Asp Ile Pro Ser Val Val
        195                 200                 205

ggg ctg cac aac gcg cgc aaa ctg att acc gag ggc gaa acg gtc att 672
Gly Leu His Asn Ala Arg Lys Leu Ile Thr Glu Gly Glu Thr Val Ile
    210                 215                 220

gtg gac ggt atc aac ggc gtg ttg att atc gcg ccg gat gag tcg gtg 720
Val Asp Gly Ile Asn Gly Val Leu Ile Ile Ala Pro Asp Glu Ser Val
225                 230                 235                 240

ttg aac gaa tac cgc cgc cgt gcc cgc gaa tac cgc agc cac aaa cgc 768
Leu Asn Glu Tyr Arg Arg Arg Ala Arg Glu Tyr Arg Ser His Lys Arg
                245                 250                 255

gat ttg aac aag ctc aaa aaa acc gcc gcc gcc acc gct gac ggg gtc 816
Asp Leu Asn Lys Leu Lys Lys Thr Ala Ala Ala Thr Ala Asp Gly Val
            260                 265                 270

tgc atc gag ctt gtg ggc aat ata gaa tcc gcc gaa gac gtg aaa ccg 864
Cys Ile Glu Leu Val Gly Asn Ile Glu Ser Ala Glu Asp Val Lys Pro
        275                 280                 285

ctg cac aac ctc ggc gca gac ggc atc ggg ctg ttc cgc agc gag ttt 912
Leu His Asn Leu Gly Ala Asp Gly Ile Gly Leu Phe Arg Ser Glu Phe
    290                 295                 300

ctt tac ctg aac cgc gat acg atg ccg tct gaa gac gag cag tac gaa 960
Leu Tyr Leu Asn Arg Asp Thr Met Pro Ser Glu Asp Glu Gln Tyr Glu
305                 310                 315                 320

gtg tac agc gcg att gtc aaa aaa atg aaa ggc aaa agc gta acg ata 1008
Val Tyr Ser Ala Ile Val Lys Lys Met Lys Gly Lys Ser Val Thr Ile
                325                 330                 335

cgg aca gtc gat ttg ggt gtg gac aaa aac ccg cgc tgg ttc ggg aaa 1056
Arg Thr Val Asp Leu Gly Val Asp Lys Asn Pro Arg Trp Phe Gly Lys
            340                 345                 350

aac agc acg ccc aac ggc agc ctc aac ccc gcg ctg ggc atg acc ggc 1104
Asn Ser Thr Pro Asn Gly Ser Leu Asn Pro Ala Leu Gly Met Thr Gly
        355                 360                 365

atc cgc ctg tgc ctt gcc gaa ccg gtc atg ttc cgc acc cag atg cgc 1152
Ile Arg Leu Cys Leu Ala Glu Pro Val Met Phe Arg Thr Gln Met Arg
    370                 375                 380

gcc atc ctc cgc gcc gcc gcc cac ggc ccc gtg cgg atg atg tgg ccg 1200
Ala Ile Leu Arg Ala Ala Ala His Gly Pro Val Arg Met Met Trp Pro
385                 390                 395                 400

atg att acc tcc gta tcc gaa gtg cgc cag tgc ctc atc cac ctc gac 1248
Met Ile Thr Ser Val Ser Glu Val Arg Gln Cys Leu Ile His Leu Asp
                405                 410                 415

acc gcg caa cgc cag ctt gcc gaa cgc ggc gat gcc ttc ggt aaa gtc     1296
Thr Ala Gln Arg Gln Leu Ala Glu Arg Gly Asp Ala Phe Gly Lys Val
            420                 425                 430

ggc atc ggc tgt atg att gaa att ccg tct gcc gcg ctg acc gtc ggc     1344
Gly Ile Gly Cys Met Ile Glu Ile Pro Ser Ala Ala Leu Thr Val Gly
        435                 440                 445

agt att ttg aaa ctg gtc gat ttc atc tcc gtc ggt acc aac gac ctg     1392
Ser Ile Leu Lys Leu Val Asp Phe Ile Ser Val Gly Thr Asn Asp Leu
    450                 455                 460

att caa tac atc ttg tcc gtc gat cgc ggc gac gac agc gtc agc cac     1440
Ile Gln Tyr Ile Leu Ser Val Asp Arg Gly Asp Asp Ser Val Ser His
465                 470                 475                 480

ctc tac cag ccc ggc cat ccc gcc gtg ctg aaa atg ctg caa cac gtc     1488
Leu Tyr Gln Pro Gly His Pro Ala Val Leu Lys Met Leu Gln His Val
                485                 490                 495

atc cgt acc gcc aac cgc atg gac aaa gac gta tcc gta tgc ggc gag     1536
Ile Arg Thr Ala Asn Arg Met Asp Lys Asp Val Ser Val Cys Gly Glu
            500                 505                 510

atg gcg ggc gat acc gcg ttt acc cgc gtt tta ttg ggt atg ggg ctg     1584
Met Ala Gly Asp Thr Ala Phe Thr Arg Val Leu Leu Gly Met Gly Leu
        515                 520                 525

cgc cgt ttt tcc atg aac ccc aac aac atc ctg ccc gtc aaa aac atc     1632
Arg Arg Phe Ser Met Asn Pro Asn Asn Ile Leu Pro Val Lys Asn Ile
    530                 535                 540

att ctg cac agc aat gtc gga cag ctc gaa agc gat att gtg aaa gtc     1680
Ile Leu His Ser Asn Val Gly Gln Leu Glu Ser Asp Ile Val Lys Val
545                 550                 555                 560

atc cgc tgc gaa gac gaa gag aag tcg gaa aag ctg atc aaa cag atg     1728
Ile Arg Cys Glu Asp Glu Glu Lys Ser Glu Lys Leu Ile Lys Gln Met
                565                 570                 575

aac agc gtg tct gtc gag gaa gaa gcc gac ttc aag ggg cgg aaa         1773
Asn Ser Val Ser Val Glu Glu Glu Ala Asp Phe Lys Gly Arg Lys
            580                 585                 590

<210> 8
<211> 591

<212> PRT
<213> Neisseria meningitidis

<400> 8
Met Ser Ile Val Leu His Gly Val Ala Ala Gly Lys Gly Ile Ala Val
1               5                   10                  15

Gly Cys Ala His Leu Ile Ala Arg Gly Thr Glu Glu Val Pro Gln Tyr
            20                  25                  30

Asp Val Ala Glu Ala Asp Thr Asp Ala Glu Ala Glu Arg Phe Asp Ala
        35                  40                  45

Ala Val Lys Ala Thr Arg Lys Glu Leu Glu Gln Leu Arg Ser Ala Ile
    50                  55                  60

Pro Glu Asn Ala Pro Thr Glu Leu Gly Ala Phe Ile Ser Leu His Leu
65                  70                  75                  80

Met Leu Leu Thr Asp Val Thr Leu Ser Arg Glu Pro Val Asp Ile Leu
                85                  90                  95

Arg Glu Gln Lys Ile Asn Ala Glu Trp Ala Leu Lys Gln Gln Ser Asp
            100                 105                 110

Lys Leu Ala Ala Gln Phe Asp Asn Met Asp Asp Ala Tyr Leu Arg Glu
        115                 120                 125

Arg Lys Gln Asp Met Leu Gln Val Val Arg Arg Ile His Asn Asn Leu
    130                 135                 140

Ile Gly Gln Gly Asn Glu Leu Glu Val Ala Asp Asn Leu Phe Asp Glu
145                 150                 155                 160

Thr Val Leu Ile Ala Asn Asp Leu Ser Pro Ala Asp Thr Val Leu Phe
                165                 170                 175

Lys Glu Gln Arg Ile Ala Ala Phe Val Thr Asp Ala Gly Gly Pro Thr
            180                 185                 190

Gly His Thr Ala Ile Leu Gly Arg Ser Leu Asp Ile Pro Ser Val Val
        195                 200                 205

Gly Leu His Asn Ala Arg Lys Leu Ile Thr Glu Gly Glu Thr Val Ile
    210                 215                 220

Val Asp Gly Ile Asn Gly Val Leu Ile Ile Ala Pro Asp Glu Ser Val
225                 230                 235                 240

Leu Asn Glu Tyr Arg Arg Arg Ala Arg Glu Tyr Arg Ser His Lys Arg
                245                 250                 255

Asp Leu Asn Lys Leu Lys Lys Thr Ala Ala Ala Thr Ala Asp Gly Val
            260                 265                 270

Cys Ile Glu Leu Val Gly Asn Ile Glu Ser Ala Glu Asp Val Lys Pro
        275                 280                 285

Leu His Asn Leu Gly Ala Asp Gly Ile Gly Leu Phe Arg Ser Glu Phe
    290                 295                 300

Leu Tyr Leu Asn Arg Asp Thr Met Pro Ser Glu Asp Glu Gln Tyr Glu
305                 310                 315                 320

Val Tyr Ser Ala Ile Val Lys Lys Met Lys Gly Lys Ser Val Thr Ile
                325                 330                 335

Arg Thr Val Asp Leu Gly Val Asp Lys Asn Pro Arg Trp Phe Gly Lys
            340                 345                 350

Asn Ser Thr Pro Asn Gly Ser Leu Asn Pro Ala Leu Gly Met Thr Gly
        355                 360                 365

Ile Arg Leu Cys Leu Ala Glu Pro Val Met Phe Arg Thr Gln Met Arg
    370                 375                 380

Ala Ile Leu Arg Ala Ala Ala His Gly Pro Val Arg Met Met Trp Pro
385                 390                 395                 400

Met Ile Thr Ser Val Ser Glu Val Arg Gln Cys Leu Ile His Leu Asp
                405                 410                 415

Thr Ala Gln Arg Gln Leu Ala Glu Arg Gly Asp Ala Phe Gly Lys Val
            420                 425                 430

Gly Ile Gly Cys Met Ile Glu Ile Pro Ser Ala Ala Leu Thr Val Gly
        435                 440                 445

Ser Ile Leu Lys Leu Val Asp Phe Ile Ser Val Gly Thr Asn Asp Leu
    450                 455                 460

Ile Gln Tyr Ile Leu Ser Val Asp Arg Gly Asp Asp Ser Val Ser His
465                 470                 475                 480

Leu Tyr Gln Pro Gly His Pro Ala Val Leu Lys Met Leu Gln His Val
                485                 490                 495

Ile Arg Thr Ala Asn Arg Met Asp Lys Asp Val Ser Val Cys Gly Glu
            500                 505                 510

Met Ala Gly Asp Thr Ala Phe Thr Arg Val Leu Leu Gly Met Gly Leu
        515                 520                 525

Arg Arg Phe Ser Met Asn Pro Asn Asn Ile Leu Pro Val Lys Asn Ile
    530                 535                 540

Ile Leu His Ser Asn Val Gly Gln Leu Glu Ser Asp Ile Val Lys Val
545                 550                 555                 560

Ile Arg Cys Glu Asp Glu Glu Lys Ser Glu Lys Leu Ile Lys Gln Met
                565                 570                 575

Asn Ser Val Ser Val Glu Glu Glu Ala Asp Phe Lys Gly Arg Lys
            580                 585                 590

<210> 9
<211> 1572
<212> DNA
<213> Neisseria meningitidis

<220>
<221> CDS
<222> (1)..(1572)

<400> 9
atg ttc aga cgg tat ctt ccg aac aga cag atg aat atg gtt tcc aaa      48
Met Phe Arg Arg Tyr Leu Pro Asn Arg Gln Met Asn Met Val Ser Lys
  1               5                  10                  15

ctg gac aaa tac tgg cag cac ccc gcc ctc tac tgg cct ttg ctc atc      96
Leu Asp Lys Tyr Trp Gln His Pro Ala Leu Tyr Trp Pro Leu Leu Ile
             20                  25                  30

ctt ttt gcc gcc gcc acc ccc ttt acc ttc gca ccc tac tac cac ttt     144
Leu Phe Ala Ala Ala Thr Pro Phe Thr Phe Ala Pro Tyr Tyr His Phe
         35                  40                  45

tgg ctg atg ccc ttg att ttc ggt gcc ttc gtc cgc ctc atc gaa ctg     192
Trp Leu Met Pro Leu Ile Phe Gly Ala Phe Val Arg Leu Ile Glu Leu
     50                  55                  60

cgt ccg cgt ttt gct gtc tct tcc gcc tac ctg ttc ggc ctg acc gca     240

Arg Pro Arg Phe Ala Val Ser Ser Ala Tyr Leu Phe Gly Leu Thr Ala
65                  70                  75                  80

tac acg aca cag ttc tac tgg ata cac acc gcc ctg cac gac gtt tcc     288
Tyr Thr Thr Gln Phe Tyr Trp Ile His Thr Ala Leu His Asp Val Ser
                85                  90                  95

ggc ctg ccc gac ctc tat gcc gta ccg ctg acc ttc cta ctc ccc gcc     336
Gly Leu Pro Asp Leu Tyr Ala Val Pro Leu Thr Phe Leu Leu Pro Ala
            100                 105                 110

tac ctt gcc ctt tat ccg gca ctg tgt ttc tgg ctg tgg aaa aaa ttt     384
Tyr Leu Ala Leu Tyr Pro Ala Leu Cys Phe Trp Leu Trp Lys Lys Phe
        115                 120                 125

acc ctg cct cgg ggc ata aaa atc ggt ttg gta ctg ccc atc ctg tgg     432
Thr Leu Pro Arg Gly Ile Lys Ile Gly Leu Val Leu Pro Ile Leu Trp
    130                 135                 140

aca ctg acc gag ttt gcc cgc gaa cgt ttc ctg acc gga ttc ggc tgg     480
Thr Leu Thr Glu Phe Ala Arg Glu Arg Phe Leu Thr Gly Phe Gly Trp
145                 150                 155                 160

ggc gca atc ggc tac tcc caa atc acc ccg gac agc ccg ctc gcc ggc     528
Gly Ala Ile Gly Tyr Ser Gln Ile Thr Pro Asp Ser Pro Leu Ala Gly
                165                 170                 175

ttt gcc cca ttt ggc ggc atc cac atg gtt aca ctg gca acc gcc ttt     576
Phe Ala Pro Phe Gly Gly Ile His Met Val Thr Leu Ala Thr Ala Phe
            180                 185                 190

ctc ggt gtc tgg ctg gtt ttg gcg agt gat aac acc gca cgt tcg ggc     624
Leu Gly Val Trp Leu Val Leu Ala Ser Asp Asn Thr Ala Arg Ser Gly
        195                 200                 205

aaa cgc ctg ctt cca att atc ctg att gcc gcc ctg ctt gcc gca ggc     672
Lys Arg Leu Leu Pro Ile Ile Leu Ile Ala Ala Leu Leu Ala Ala Gly
    210                 215                 220

tac acc gcc cga caa acc gac ttc acc cgc ccc gac ggc agc cgc agc     720
Tyr Thr Ala Arg Gln Thr Asp Phe Thr Arg Pro Asp Gly Ser Arg Ser
225                 230                 235                 240

acc gtc gcc ctg ctt caa ggc aac atc gac caa acc ctc aaa tgg cgt     768
Thr Val Ala Leu Leu Gln Gly Asn Ile Asp Gln Thr Leu Lys Trp Arg
                245                 250                 255

gaa gac caa gtc atc ccg acc ata cag aaa tat tac gaa cag gtc ggc     816

Glu Asp Gln Val Ile Pro Thr Ile Gln Lys Tyr Tyr Glu Gln Val Gly
            260                 265                 270

aaa acc act gcc gac atc gtc atc ctg ccc gaa acc gcc atc ccc gtc 864
Lys Thr Thr Ala Asp Ile Val Ile Leu Pro Glu Thr Ala Ile Pro Val
        275                 280                 285

atg cgc caa aac ctg ccg gaa aac ata ctg gca aaa ttt gcc gaa cag 912
Met Arg Gln Asn Leu Pro Glu Asn Ile Leu Ala Lys Phe Ala Glu Gln
    290                 295                 300

gcg caa aac aac ggc agc gcg ctc gcc gtc ggc atc agc caa tac act 960
Ala Gln Asn Asn Gly Ser Ala Leu Ala Val Gly Ile Ser Gln Tyr Thr
305                 310                 315                 320

tcg gac ggc aac ggt tac gaa aac gcc gtc atc aac ctg acc ggt tat 1008
Ser Asp Gly Asn Gly Tyr Glu Asn Ala Val Ile Asn Leu Thr Gly Tyr
                325                 330                 335

cag gaa aac aat cag gac ggc atc ccc tac tac gcc aaa aac cac ctc 1056
Gln Glu Asn Asn Gln Asp Gly Ile Pro Tyr Tyr Ala Lys Asn His Leu
            340                 345                 350

gtc ccc ttc ggc gaa tac aaa ccg ctg ccc ttc ctg acc acg ccg ctt 1104
Val Pro Phe Gly Glu Tyr Lys Pro Leu Pro Phe Leu Thr Thr Pro Leu
        355                 360                 365

tac aaa atg atg aat atg ccc ctt tcc gac ttc cgc aaa ggc ggc ggt 1152
Tyr Lys Met Met Asn Met Pro Leu Ser Asp Phe Arg Lys Gly Gly Gly
    370                 375                 380

aag caa tcc gcc ctg ctg atg aaa aac caa aaa atc gcc ttc aac atc 1200
Lys Gln Ser Ala Leu Leu Met Lys Asn Gln Lys Ile Ala Phe Asn Ile
385                 390                 395                 400

tgt tac gaa gac gga ttc ggc gac gaa ctg att gcc gcc gcc aaa gat 1248
Cys Tyr Glu Asp Gly Phe Gly Asp Glu Leu Ile Ala Ala Ala Lys Asp
                405                 410                 415

gcc aca ctg ctt gcc aat gcc agc aat atg gcg tgg tac gga aaa tcc 1296
Ala Thr Leu Leu Ala Asn Ala Ser Asn Met Ala Trp Tyr Gly Lys Ser
            420                 425                 430

aac gcc atg tac cag cac ctc caa caa tcg cag gcg cgg gct atg gaa 1344
Asn Ala Met Tyr Gln His Leu Gln Gln Ser Gln Ala Arg Ala Met Glu
        435                 440                 445

ctc gga cgc tat atg gtc cgt gcc acc aac acc ggc gca acc gcc atc 1392

Leu Gly Arg Tyr Met Val Arg Ala Thr Asn Thr Gly Ala Thr Ala Ile
    450                 455                 460

atc tcc ccc aaa ggc aac atc atc gcc caa gcc caa ccc gat acg gaa    1440
Ile Ser Pro Lys Gly Asn Ile Ile Ala Gln Ala Gln Pro Asp Thr Glu
465                 470                 475                 480

acc gta ttg gaa gga cac atc aaa ggc tat gtc ggc gaa acg ccc tat    1488
Thr Val Leu Glu Gly His Ile Lys Gly Tyr Val Gly Glu Thr Pro Tyr
                485                 490                 495

atg aaa acc ggc agt tca tgg tgg ttg atg ggc ata ttg acc cta gcc    1536
Met Lys Thr Gly Ser Ser Trp Trp Leu Met Gly Ile Leu Thr Leu Ala
            500                 505                 510

gca ctg att ctt ttc atc ttc cga aac aaa gaa cac                    1572
Ala Leu Ile Leu Phe Ile Phe Arg Asn Lys Glu His
        515                 520

<210> 10
<211> 524
<212> PRT
<213> Neisseria meningitidis

<400> 10
Met Phe Arg Arg Tyr Leu Pro Asn Arg Gln Met Asn Met Val Ser Lys
1               5                   10                  15

Leu Asp Lys Tyr Trp Gln His Pro Ala Leu Tyr Trp Pro Leu Leu Ile
            20                  25                  30

Leu Phe Ala Ala Ala Thr Pro Phe Thr Phe Ala Pro Tyr Tyr His Phe
        35                  40                  45

Trp Leu Met Pro Leu Ile Phe Gly Ala Phe Val Arg Leu Ile Glu Leu
    50                  55                  60

Arg Pro Arg Phe Ala Val Ser Ser Ala Tyr Leu Phe Gly Leu Thr Ala
65                  70                  75                  80

Tyr Thr Thr Gln Phe Tyr Trp Ile His Thr Ala Leu His Asp Val Ser
                85                  90                  95

Gly Leu Pro Asp Leu Tyr Ala Val Pro Leu Thr Phe Leu Leu Pro Ala
            100                 105                 110

Tyr Leu Ala Leu Tyr Pro Ala Leu Cys Phe Trp Leu Trp Lys Lys Phe

        115                 120                 125

Thr Leu Pro Arg Gly Ile Lys Ile Gly Leu Val Leu Pro Ile Leu Trp
    130                 135                 140

Thr Leu Thr Glu Phe Ala Arg Glu Arg Phe Leu Thr Gly Phe Gly Trp
145                 150                 155                 160

Gly Ala Ile Gly Tyr Ser Gln Ile Thr Pro Asp Ser Pro Leu Ala Gly
                165                 170                 175

Phe Ala Pro Phe Gly Gly Ile His Met Val Thr Leu Ala Thr Ala Phe
            180                 185                 190

Leu Gly Val Trp Leu Val Leu Ala Ser Asp Asn Thr Ala Arg Ser Gly
        195                 200                 205

Lys Arg Leu Leu Pro Ile Ile Leu Ile Ala Ala Leu Leu Ala Ala Gly
    210                 215                 220

Tyr Thr Ala Arg Gln Thr Asp Phe Thr Arg Pro Asp Gly Ser Arg Ser
225                 230                 235                 240

Thr Val Ala Leu Leu Gln Gly Asn Ile Asp Gln Thr Leu Lys Trp Arg
                245                 250                 255

Glu Asp Gln Val Ile Pro Thr Ile Gln Lys Tyr Tyr Glu Gln Val Gly
            260                 265                 270

Lys Thr Thr Ala Asp Ile Val Ile Leu Pro Glu Thr Ala Ile Pro Val
        275                 280                 285

Met Arg Gln Asn Leu Pro Glu Asn Ile Leu Ala Lys Phe Ala Glu Gln
    290                 295                 300

Ala Gln Asn Asn Gly Ser Ala Leu Ala Val Gly Ile Ser Gln Tyr Thr
305                 310                 315                 320

Ser Asp Gly Asn Gly Tyr Glu Asn Ala Val Ile Asn Leu Thr Gly Tyr
                325                 330                 335

Gln Glu Asn Asn Gln Asp Gly Ile Pro Tyr Tyr Ala Lys Asn His Leu
            340                 345                 350

Val Pro Phe Gly Glu Tyr Lys Pro Leu Pro Phe Leu Thr Thr Pro Leu
        355                 360                 365

Tyr Lys Met Met Asn Met Pro Leu Ser Asp Phe Arg Lys Gly Gly Gly

370                 375                 380

Lys Gln Ser Ala Leu Leu Met Lys Asn Gln Lys Ile Ala Phe Asn Ile
385                 390                 395                 400

Cys Tyr Glu Asp Gly Phe Gly Asp Glu Leu Ile Ala Ala Ala Lys Asp
                405                 410                 415

Ala Thr Leu Leu Ala Asn Ala Ser Asn Met Ala Trp Tyr Gly Lys Ser
            420                 425                 430

Asn Ala Met Tyr Gln His Leu Gln Gln Ser Gln Ala Arg Ala Met Glu
        435                 440                 445

Leu Gly Arg Tyr Met Val Arg Ala Thr Asn Thr Gly Ala Thr Ala Ile
    450                 455                 460

Ile Ser Pro Lys Gly Asn Ile Ile Ala Gln Ala Gln Pro Asp Thr Glu
465                 470                 475                 480

Thr Val Leu Glu Gly His Ile Lys Gly Tyr Val Gly Glu Thr Pro Tyr
                485                 490                 495

Met Lys Thr Gly Ser Ser Trp Trp Leu Met Gly Ile Leu Thr Leu Ala
            500                 505                 510

Ala Leu Ile Leu Phe Ile Phe Arg Asn Lys Glu His
        515                 520

<210> 11
<211> 1185
<212> DNA
<213> Neisseria meningitidis

<220>
<221> CDS
<222> (1)..(1185)

<400> 11
atg aca gag gac gaa cgt ttc gcg tgg ctg caa ttg gcg ttt acg ccc      48
Met Thr Glu Asp Glu Arg Phe Ala Trp Leu Gln Leu Ala Phe Thr Pro
1               5                   10                  15

tat atc ggc gcg gaa agt ttc ctg ctg ctg atg cgc cgt ttc ggc agc      96
Tyr Ile Gly Ala Glu Ser Phe Leu Leu Leu Met Arg Arg Phe Gly Ser
            20                  25                  30

gcg caa aat gcc ctg tcc gca ccg gcg gaa cag gtg gcg gca ctg ata 144
Ala Gln Asn Ala Leu Ser Ala Pro Ala Glu Gln Val Ala Ala Leu Ile
        35                  40                  45

cgg cac aaa cag gcg ctt gag gct tgg cgc aat gcg gaa aaa cgc gct 192
Arg His Lys Gln Ala Leu Glu Ala Trp Arg Asn Ala Glu Lys Arg Ala
    50                  55                  60

ctg gcg cgg cag gcg gca gaa gcg gcg ttg gaa tgg gaa atg cgg gac 240
Leu Ala Arg Gln Ala Ala Glu Ala Ala Leu Glu Trp Glu Met Arg Asp
65                  70                  75                  80

gga tgc cgc ctg atg ctg ctt cag gat gaa gat ttc ccc gaa atg ctg 288
Gly Cys Arg Leu Met Leu Leu Gln Asp Glu Asp Phe Pro Glu Met Leu
                85                  90                  95

acg cag ggg ctg acc gcg ccg ccg gtt ttg ttt ttg cgc ggc aac gtg 336
Thr Gln Gly Leu Thr Ala Pro Pro Val Leu Phe Leu Arg Gly Asn Val
            100                 105                 110

cgg ctg ctg cac aag cct tcc gcc gcc atc gtc ggc agc cgc cat gcc 384
Arg Leu Leu His Lys Pro Ser Ala Ala Ile Val Gly Ser Arg His Ala
        115                 120                 125

acg ccg cag gcg atg cgg att gcc aaa gat ttc ggc aag tcg ttg ggc 432
Thr Pro Gln Ala Met Arg Ile Ala Lys Asp Phe Gly Lys Ser Leu Gly
    130                 135                 140

ggg caa aat atc ccc gtt gtg tcg ggt atg gct tcc ggt atc gat act 480
Gly Gln Asn Ile Pro Val Val Ser Gly Met Ala Ser Gly Ile Asp Thr
145                 150                 155                 160

gcc gcc cat cag ggc gca ctg gag gca gaa ggc ggc acc atc gcc gtg 528
Ala Ala His Gln Gly Ala Leu Glu Ala Glu Gly Gly Thr Ile Ala Val
                165                 170                 175

tgg ggg acg ggt ata gac cgc att tac ccg ccg tcc aat aaa aac ctt 576
Trp Gly Thr Gly Ile Asp Arg Ile Tyr Pro Pro Ser Asn Lys Asn Leu
            180                 185                 190

gcc tat gaa atc gcc gaa aaa gga ttg att gtc agc gag ttt ccc atc 624
Ala Tyr Glu Ile Ala Glu Lys Gly Leu Ile Val Ser Glu Phe Pro Ile
        195                 200                 205

ggt acg cgc ccg tat gcc ggc aat ttt ccg cgc cgc aac cgc ctg att 672
Gly Thr Arg Pro Tyr Ala Gly Asn Phe Pro Arg Arg Asn Arg Leu Ile
    210                 215                 220

gcc gcc ctg tcg caa gta acg ctg gtg gtt gaa gcc gcc ttg gaa tcc     720
Ala Ala Leu Ser Gln Val Thr Leu Val Val Glu Ala Ala Leu Glu Ser
225                 230                 235                 240

ggt tcg ctg att acc gcc ggc ctg gcg gca gaa atg ggg cgc gaa gtg     768
Gly Ser Leu Ile Thr Ala Gly Leu Ala Ala Glu Met Gly Arg Glu Val
                245                 250                 255

atg gcg gta ccc ggt tcg ata gac aat ccg cac agc aaa ggc tgc cac     816
Met Ala Val Pro Gly Ser Ile Asp Asn Pro His Ser Lys Gly Cys His
            260                 265                 270

aaa ctg att aaa gac ggc gca aaa ttg gtg gaa tgc ctg gac gac atc     864
Lys Leu Ile Lys Asp Gly Ala Lys Leu Val Glu Cys Leu Asp Asp Ile
        275                 280                 285

ctg aac gaa tgc ccg ggg cta ttg caa aat acg ggt gct tca tca tat     912
Leu Asn Glu Cys Pro Gly Leu Leu Gln Asn Thr Gly Ala Ser Ser Tyr
    290                 295                 300

tct ata aat aag gat acg ccc gac acg gga cgc cgc gcc gtt cag acg     960
Ser Ile Asn Lys Asp Thr Pro Asp Thr Gly Arg Arg Ala Val Gln Thr
305                 310                 315                 320

gca tac gcc ccg ccg cct gcc gca aaa atg ccg tct gaa gcg gcg gca     1008
Ala Tyr Ala Pro Pro Pro Ala Ala Lys Met Pro Ser Glu Ala Ala Ala
                325                 330                 335

ggc ggc aca gcc gtc ggc ggc ata ctg gat aaa atg ggt ttc gac ccc     1056
Gly Gly Thr Ala Val Gly Gly Ile Leu Asp Lys Met Gly Phe Asp Pro
            340                 345                 350

atc cat ccc gac gtg ctt gcc gga cag ttg gct atg cct gcc gca gat     1104
Ile His Pro Asp Val Leu Ala Gly Gln Leu Ala Met Pro Ala Ala Asp
        355                 360                 365

ttg tat gcc gca ctg ttg gaa ttg gaa ttg gac ggc agc gtt gcc gca     1152
Leu Tyr Ala Ala Leu Leu Glu Leu Glu Leu Asp Gly Ser Val Ala Ala
    370                 375                 380

atg ccc agc ggc aga tac cag cgt atc cga act                         1185
Met Pro Ser Gly Arg Tyr Gln Arg Ile Arg Thr
385                 390                 395

<210> 12
<211> 395

<212> PRT
<213> Neisseria meningitidis

<400> 12
Met Thr Glu Asp Glu Arg Phe Ala Trp Leu Gln Leu Ala Phe Thr Pro
1               5                   10                  15

Tyr Ile Gly Ala Glu Ser Phe Leu Leu Leu Met Arg Arg Phe Gly Ser
            20                  25                  30

Ala Gln Asn Ala Leu Ser Ala Pro Ala Glu Gln Val Ala Ala Leu Ile
        35                  40                  45

Arg His Lys Gln Ala Leu Glu Ala Trp Arg Asn Ala Glu Lys Arg Ala
    50                  55                  60

Leu Ala Arg Gln Ala Ala Glu Ala Ala Leu Glu Trp Glu Met Arg Asp
65                  70                  75                  80

Gly Cys Arg Leu Met Leu Leu Gln Asp Glu Asp Phe Pro Glu Met Leu
                85                  90                  95

Thr Gln Gly Leu Thr Ala Pro Pro Val Leu Phe Leu Arg Gly Asn Val
            100                 105                 110

Arg Leu Leu His Lys Pro Ser Ala Ala Ile Val Gly Ser Arg His Ala
        115                 120                 125

Thr Pro Gln Ala Met Arg Ile Ala Lys Asp Phe Gly Lys Ser Leu Gly
    130                 135                 140

Gly Gln Asn Ile Pro Val Val Ser Gly Met Ala Ser Gly Ile Asp Thr
145                 150                 155                 160

Ala Ala His Gln Gly Ala Leu Glu Ala Glu Gly Gly Thr Ile Ala Val
                165                 170                 175

Trp Gly Thr Gly Ile Asp Arg Ile Tyr Pro Pro Ser Asn Lys Asn Leu
            180                 185                 190

Ala Tyr Glu Ile Ala Glu Lys Gly Leu Ile Val Ser Glu Phe Pro Ile
        195                 200                 205

Gly Thr Arg Pro Tyr Ala Gly Asn Phe Pro Arg Arg Asn Arg Leu Ile
    210                 215                 220

Ala Ala Leu Ser Gln Val Thr Leu Val Val Glu Ala Ala Leu Glu Ser
225                 230                 235                 240

Gly Ser Leu Ile Thr Ala Gly Leu Ala Ala Glu Met Gly Arg Glu Val
                245                 250                 255

Met Ala Val Pro Gly Ser Ile Asp Asn Pro His Ser Lys Gly Cys His
            260                 265                 270

Lys Leu Ile Lys Asp Gly Ala Lys Leu Val Glu Cys Leu Asp Asp Ile
        275                 280                 285

Leu Asn Glu Cys Pro Gly Leu Leu Gln Asn Thr Gly Ala Ser Ser Tyr
    290                 295                 300

Ser Ile Asn Lys Asp Thr Pro Asp Thr Gly Arg Arg Ala Val Gln Thr
305                 310                 315                 320

Ala Tyr Ala Pro Pro Pro Ala Ala Lys Met Pro Ser Glu Ala Ala Ala
                325                 330                 335

Gly Gly Thr Ala Val Gly Gly Ile Leu Asp Lys Met Gly Phe Asp Pro
            340                 345                 350

Ile His Pro Asp Val Leu Ala Gly Gln Leu Ala Met Pro Ala Ala Asp
        355                 360                 365

Leu Tyr Ala Ala Leu Leu Glu Leu Glu Leu Asp Gly Ser Val Ala Ala
    370                 375                 380

Met Pro Ser Gly Arg Tyr Gln Arg Ile Arg Thr
385                 390                 395

<210> 13
<211> 804
<212> DNA
<213> Neisseria meningitidis

<220>
<221> CDS
<222> (1)..(804)

<400> 13
atg ccg aca gtt ttc atc ttt ttg acg gcg gtt tac gca gga ttg ggt      48
Met Pro Thr Val Phe Ile Phe Leu Thr Ala Val Tyr Ala Gly Leu Gly
  1               5                  10                  15

gca ttt gca tgg cac tgc caa cag cag ggg ctc ggc cgg gat tac ccg      96

Ala Phe Ala Trp His Cys Gln Gln Gln Gly Leu Gly Arg Asp Tyr Pro
            20                  25                  30

tgg aag acg gaa ttg ccg gtt ttg ggc gcg gca ttg acc gtc cat ggc 144
Trp Lys Thr Glu Leu Pro Val Leu Gly Ala Ala Leu Thr Val His Gly
        35                  40                  45

gcg gca ctg ctt atg ccg gtc att caa gac aaa atc atc att atg ggc 192
Ala Ala Leu Leu Met Pro Val Ile Gln Asp Lys Ile Ile Ile Met Gly
    50                  55                  60

ttt ggg tat tcc ggc agc ctg att gtt tgg atg atg ctg ttt att tat 240
Phe Gly Tyr Ser Gly Ser Leu Ile Val Trp Met Met Leu Phe Ile Tyr
65                  70                  75                  80

ttt gcc ggc agc ttc ttt tat tcg ctg cgc gga gtg cag ttg ctg ctg 288
Phe Ala Gly Ser Phe Phe Tyr Ser Leu Arg Gly Val Gln Leu Leu Leu
                85                  90                  95

tat cct tgc gcc gca ctg atg ctg ctg tca ggt ttg gtt ttt cct gga 336
Tyr Pro Cys Ala Ala Leu Met Leu Leu Ser Gly Leu Val Phe Pro Gly
            100                 105                 110

aaa ttc tcg gga tat gaa att acc gac ctt ccc ttt atg ctg cat atc 384
Lys Phe Ser Gly Tyr Glu Ile Thr Asp Leu Pro Phe Met Leu His Ile
        115                 120                 125

gga act tcg ctg ctt gca tac ggg ctg ttc ggc atc gca aca tta ttg 432
Gly Thr Ser Leu Leu Ala Tyr Gly Leu Phe Gly Ile Ala Thr Leu Leu
    130                 135                 140

tcc gtt ttg acc ctg ctg ctg aat cgg agc ctg cac cgc agg aac ttc 480
Ser Val Leu Thr Leu Leu Leu Asn Arg Ser Leu His Arg Arg Asn Phe
145                 150                 155                 160

tcc aag ctc gca gga ttc ctg ccg tcg ctg ctc agt ttg gaa aaa ctc 528
Ser Lys Leu Ala Gly Phe Leu Pro Ser Leu Leu Ser Leu Glu Lys Leu
                165                 170                 175

atg ttc cag gcc atg tgg gca ggt ttc atc ctg ctg acc tat tcc gtc 576
Met Phe Gln Ala Met Trp Ala Gly Phe Ile Leu Leu Thr Tyr Ser Val
            180                 185                 190

gtc agc gga aca ttt ttt gcc gaa gcc gta ttc ggc aaa ccc atg acc 624
Val Ser Gly Thr Phe Phe Ala Glu Ala Val Phe Gly Lys Pro Met Thr
        195                 200                 205

ttt acc cat aaa acc gta ttc ggc ata ttg tca tgg ctg att tac ggc 672

Phe Thr His Lys Thr Val Phe Gly Ile Leu Ser Trp Leu Ile Tyr Gly
    210                 215                 220

gga ctg ctg ctc aag cac agc atg acc gca tgg cgc ggc aaa aaa gcc     720
Gly Leu Leu Leu Lys His Ser Met Thr Ala Trp Arg Gly Lys Lys Ala
225                 230                 235                 240

gcc gtg tgg acc atc atc gga ttt gtc agc ctg atg att gcc tat atg     768
Ala Val Trp Thr Ile Ile Gly Phe Val Ser Leu Met Ile Ala Tyr Met
                245                 250                 255

ggc agc aag ttc gta ttg gaa atc att ctg aac aga                     804
Gly Ser Lys Phe Val Leu Glu Ile Ile Leu Asn Arg
            260                 265

<210> 14
<211> 268
<212> PRT
<213> Neisseria meningitidis

<400> 14
Met Pro Thr Val Phe Ile Phe Leu Thr Ala Val Tyr Ala Gly Leu Gly
  1               5                  10                  15

Ala Phe Ala Trp His Cys Gln Gln Gln Gly Leu Gly Arg Asp Tyr Pro
             20                  25                  30

Trp Lys Thr Glu Leu Pro Val Leu Gly Ala Ala Leu Thr Val His Gly
         35                  40                  45

Ala Ala Leu Leu Met Pro Val Ile Gln Asp Lys Ile Ile Ile Met Gly
     50                  55                  60

Phe Gly Tyr Ser Gly Ser Leu Ile Val Trp Met Met Leu Phe Ile Tyr
 65                  70                  75                  80

Phe Ala Gly Ser Phe Phe Tyr Ser Leu Arg Gly Val Gln Leu Leu Leu
                 85                  90                  95

Tyr Pro Cys Ala Ala Leu Met Leu Leu Ser Gly Leu Val Phe Pro Gly
            100                 105                 110

Lys Phe Ser Gly Tyr Glu Ile Thr Asp Leu Pro Phe Met Leu His Ile
        115                 120                 125

Gly Thr Ser Leu Leu Ala Tyr Gly Leu Phe Gly Ile Ala Thr Leu Leu
    130                 135                 140

Ser Val Leu Thr Leu Leu Leu Asn Arg Ser Leu His Arg Arg Asn Phe
145                 150                 155                 160

Ser Lys Leu Ala Gly Phe Leu Pro Ser Leu Leu Ser Leu Glu Lys Leu
                165                 170                 175

Met Phe Gln Ala Met Trp Ala Gly Phe Ile Leu Leu Thr Tyr Ser Val
            180                 185                 190

Val Ser Gly Thr Phe Phe Ala Glu Ala Val Phe Gly Lys Pro Met Thr
        195                 200                 205

Phe Thr His Lys Thr Val Phe Gly Ile Leu Ser Trp Leu Ile Tyr Gly
    210                 215                 220

Gly Leu Leu Leu Lys His Ser Met Thr Ala Trp Arg Gly Lys Lys Ala
225                 230                 235                 240

Ala Val Trp Thr Ile Ile Gly Phe Val Ser Leu Met Ile Ala Tyr Met
                245                 250                 255

Gly Ser Lys Phe Val Leu Glu Ile Ile Leu Asn Arg
            260                 265

<210> 15
<211> 2391
<212> DNA
<213> Neisseria meningitidis

<220>
<221> CDS
<222> (1)..(2391)

<400> 15
atg tca aca gtc caa atg aat aaa aat att aaa tct tta aat tta cgg     48
Met Ser Thr Val Gln Met Asn Lys Asn Ile Lys Ser Leu Asn Leu Arg
  1               5                  10                  15

gaa aaa gac ccg ttt tta agt cgt gaa aaa cag cgt tat gaa cat cct     96
Glu Lys Asp Pro Phe Leu Ser Arg Glu Lys Gln Arg Tyr Glu His Pro
             20                  25                  30

ttg ccc agt cgg gaa tgg ata atc gag ctg ctt gaa cgt aaa ggc gta    144
Leu Pro Ser Arg Glu Trp Ile Ile Glu Leu Leu Glu Arg Lys Gly Val
         35                  40                  45

cca tcc aag att gaa gct ttg gta cgc gaa ttg tcg att aag gaa gaa    192
Pro Ser Lys Ile Glu Ala Leu Val Arg Glu Leu Ser Ile Lys Glu Glu
     50                  55                  60

gag tac gaa ttt ttc gaa cgt cgt ctg aag gcg atg gcg cgg gac ggt    240
Glu Tyr Glu Phe Phe Glu Arg Arg Leu Lys Ala Met Ala Arg Asp Gly
 65                  70                  75                  80

cag gtt tta atc aac cgt cgg ggc gcg gtt tgc gcg gcg gac aaa ttg    288
Gln Val Leu Ile Asn Arg Arg Gly Ala Val Cys Ala Ala Asp Lys Leu
                 85                  90                  95

gat ttg gtc aaa tgc cgt gtc gag gcg cac aaa gac ggc ttc ggt ttc    336
Asp Leu Val Lys Cys Arg Val Glu Ala His Lys Asp Gly Phe Gly Phe
            100                 105                 110

gcc gtg ccg ctc acg ccc gcc aaa gac ggt gat ttt gtt ttg tac gaa    384
Ala Val Pro Leu Thr Pro Ala Lys Asp Gly Asp Phe Val Leu Tyr Glu
        115                 120                 125

cgc cag atg cgc ggc att atg cac ggc gat att gtc act gtt cgt cct    432
Arg Gln Met Arg Gly Ile Met His Gly Asp Ile Val Thr Val Arg Pro
    130                 135                 140

gcc ggc atg gac cgt agg ggc cgc cgc gaa ggg acg gtt ctg gat att    480
Ala Gly Met Asp Arg Arg Gly Arg Arg Glu Gly Thr Val Leu Asp Ile
145                 150                 155                 160

gtc gaa cgc gcg caa agc aaa gtg gtc ggc cgt ttc tat atg gat agg    528
Val Glu Arg Ala Gln Ser Lys Val Val Gly Arg Phe Tyr Met Asp Arg
                165                 170                 175

ggc gtg gcg att ttg gag ccg gaa gac aag cgt ctg aac caa agc atc    576
Gly Val Ala Ile Leu Glu Pro Glu Asp Lys Arg Leu Asn Gln Ser Ile
            180                 185                 190

gta ttg gaa ccg gac ggc gtg gcg cgt ttc aaa cct gaa tcc ggt cag    624
Val Leu Glu Pro Asp Gly Val Ala Arg Phe Lys Pro Glu Ser Gly Gln
        195                 200                 205

gtc atc gtc ggc gaa att gag gtt tat cct gag caa aac cgg ccg gca    672
Val Ile Val Gly Glu Ile Glu Val Tyr Pro Glu Gln Asn Arg Pro Ala
    210                 215                 220

gtg gca aaa atc atc gaa gtt ttg ggc gat tat gcc gac agc ggc atg    720
Val Ala Lys Ile Ile Glu Val Leu Gly Asp Tyr Ala Asp Ser Gly Met
225                 230                 235                 240

gag att gaa att gcc gtg cgc aag cat cat ttg ccg cac caa ttc agt 768
Glu Ile Glu Ile Ala Val Arg Lys His His Leu Pro His Gln Phe Ser
                245                 250                 255

gaa gcg tgt gcc aaa gcc gcg aaa aaa att ccc gac cat gta cgc aaa 816
Glu Ala Cys Ala Lys Ala Ala Lys Lys Ile Pro Asp His Val Arg Lys
            260                 265                 270

agc gat ttg aaa ggc cgc gtc gat ttg cgc gac ctg cct ttg gta acg 864
Ser Asp Leu Lys Gly Arg Val Asp Leu Arg Asp Leu Pro Leu Val Thr
        275                 280                 285

ata gac ggc gaa acg gct cga gat ttt gac gat gcg gtg ttt gcc gag 912
Ile Asp Gly Glu Thr Ala Arg Asp Phe Asp Asp Ala Val Phe Ala Glu
    290                 295                 300

aaa atc gga cgc aat tac cgt ctg gtc gtg gcg att gcc gat gtc agc 960
Lys Ile Gly Arg Asn Tyr Arg Leu Val Val Ala Ile Ala Asp Val Ser
305                 310                 315                 320

cat tat gtc cgc ccc gat gac gct atc gac acg gac gct cag gaa cgc 1008
His Tyr Val Arg Pro Asp Asp Ala Ile Asp Thr Asp Ala Gln Glu Arg
                325                 330                 335

agc acc agt gtt tac ttc ccg cgc cgc gtg att ccc atg ttg ccg gaa 1056
Ser Thr Ser Val Tyr Phe Pro Arg Arg Val Ile Pro Met Leu Pro Glu
            340                 345                 350

aac ctg tcc aac ggc atc tgc tcg ctc aat cct cat gtc gag cgt ttg 1104
Asn Leu Ser Asn Gly Ile Cys Ser Leu Asn Pro His Val Glu Arg Leu
        355                 360                 365

tgt gtg gtg tgc gat atg gtt atc act tac gcg ggc aat atc aaa gaa 1152
Cys Val Val Cys Asp Met Val Ile Thr Tyr Ala Gly Asn Ile Lys Glu
    370                 375                 380

tac cgc ttc tac ccc gcc gtg atg cgc tct cat gcc cgc ctg acc tac 1200
Tyr Arg Phe Tyr Pro Ala Val Met Arg Ser His Ala Arg Leu Thr Tyr
385                 390                 395                 400

aac caa gtt tgg aaa tgg ctt tca ggc ggc atc gag cat ccg ttc aaa 1248
Asn Gln Val Trp Lys Trp Leu Ser Gly Gly Ile Glu His Pro Phe Lys
                405                 410                 415

acc caa atc gac acg ctt tac aaa ctc ttc aaa atc ctt cag aaa aag 1296
Thr Gln Ile Asp Thr Leu Tyr Lys Leu Phe Lys Ile Leu Gln Lys Lys
            420                 425                 430

cgt ttc gaa cgc ggg gcg gtg gag ttt gac agc atc gaa acc caa atg 1344
Arg Phe Glu Arg Gly Ala Val Glu Phe Asp Ser Ile Glu Thr Gln Met
        435                 440                 445

ctt ttc gac gac aac ggt aaa att gaa aaa atc gtc ccc gtt gtc cgc 1392
Leu Phe Asp Asp Asn Gly Lys Ile Glu Lys Ile Val Pro Val Val Arg
    450                 455                 460

aac gat gcc cac aag ctg att gaa gaa tgt atg ttg gcg gca aac gtt 1440
Asn Asp Ala His Lys Leu Ile Glu Glu Cys Met Leu Ala Ala Asn Val
465                 470                 475                 480

tgc gca gcg gat ttt ctg ttg aaa aac aag cat acc gca ttg ttc cgc 1488
Cys Ala Ala Asp Phe Leu Leu Lys Asn Lys His Thr Ala Leu Phe Arg
                485                 490                 495

aac cat ttg ggg ccc acg ccc gaa aaa ctc gcc gcc ttg cgc gag cag 1536
Asn His Leu Gly Pro Thr Pro Glu Lys Leu Ala Ala Leu Arg Glu Gln
            500                 505                 510

ctc ggt ctg ttg ggg ctt caa ctt ggc ggc ggc gac aac ccg tcg ccg 1584
Leu Gly Leu Leu Gly Leu Gln Leu Gly Gly Gly Asp Asn Pro Ser Pro
        515                 520                 525

aaa gac tat gcc gcg ctt gcc gga cag ttc aaa ggc agg ccg gat gcc 1632
Lys Asp Tyr Ala Ala Leu Ala Gly Gln Phe Lys Gly Arg Pro Asp Ala
    530                 535                 540

gaa ttg ctg caa gtc atg atg ttg cgc tcc atg caa cag gcg gtt tac 1680
Glu Leu Leu Gln Val Met Met Leu Arg Ser Met Gln Gln Ala Val Tyr
545                 550                 555                 560

gaa ccg cat tgc gac gga cac ttt ggt ctt gcc tac gaa gca tac gcc 1728
Glu Pro His Cys Asp Gly His Phe Gly Leu Ala Tyr Glu Ala Tyr Ala
                565                 570                 575

cac ttc acc tcg ccc atc cgc cgc tat ccc gac ctg acc gta cac cgc 1776
His Phe Thr Ser Pro Ile Arg Arg Tyr Pro Asp Leu Thr Val His Arg
            580                 585                 590

gcc atc aaa gcc gtg ttg aat cag caa acc tac acg cca aaa aaa agc 1824
Ala Ile Lys Ala Val Leu Asn Gln Gln Thr Tyr Thr Pro Lys Lys Ser
        595                 600                 605

tgg cag gct ttg ggc gtg cat acc tcg ttc tgt gag cgc cgt gcc gac 1872
Trp Gln Ala Leu Gly Val His Thr Ser Phe Cys Glu Arg Arg Ala Asp
    610                 615                 620

gac gcc agc cgc gac gtg gaa aac tgg ctg aaa acc tat tat atg cgc 1920
Asp Ala Ser Arg Asp Val Glu Asn Trp Leu Lys Thr Tyr Tyr Met Arg
625                 630                 635                 640

gat aag gtc ggc gaa gta ttc gaa ggt aaa atc tcc ggc atg acc agt 1968
Asp Lys Val Gly Glu Val Phe Glu Gly Lys Ile Ser Gly Met Thr Ser
                645                 650                 655

ttt ggt atc ttt gta aca ctg gac ggc atc cac att gac ggc ttg gtg 2016
Phe Gly Ile Phe Val Thr Leu Asp Gly Ile His Ile Asp Gly Leu Val
            660                 665                 670

cat atc agc gat ttg ggc gaa gac tat ttc aac ttc cgc ccc gaa atc 2064
His Ile Ser Asp Leu Gly Glu Asp Tyr Phe Asn Phe Arg Pro Glu Ile
        675                 680                 685

atg gca atc gaa ggc gaa cgc agc ggc atc cgt ttc aac atg ggg gac 2112
Met Ala Ile Glu Gly Glu Arg Ser Gly Ile Arg Phe Asn Met Gly Asp
    690                 695                 700

agg gtt gcc gtc cgg gtc gcc cgt gcc gat ttg gat gac gga aaa atc 2160
Arg Val Ala Val Arg Val Ala Arg Ala Asp Leu Asp Asp Gly Lys Ile
705                 710                 715                 720

gat ttt gtc ctg att gcc ggg ggg agc ggc agg ggg cgg aaa gtt aaa 2208
Asp Phe Val Leu Ile Ala Gly Gly Ser Gly Arg Gly Arg Lys Val Lys
                725                 730                 735

tca tcc gcg tct gcc aaa ccg gca ggg acg gcg ggg aaa ggg aag ccg 2256
Ser Ser Ala Ser Ala Lys Pro Ala Gly Thr Ala Gly Lys Gly Lys Pro
            740                 745                 750

aaa acc gcc gcc gag aaa aaa aca gcc cga ggc ggc aaa gta agg gga 2304
Lys Thr Ala Ala Glu Lys Lys Thr Ala Arg Gly Gly Lys Val Arg Gly
        755                 760                 765

agg ggc gcg tct gcc gcc gca gaa tcg agg aaa aag gca aag aaa ccg 2352
Arg Gly Ala Ser Ala Ala Ala Glu Ser Arg Lys Lys Ala Lys Lys Pro
    770                 775                 780

gtt ccg att aag gta aaa aaa cgg aaa ggc aaa tca taa 2391
Val Pro Ile Lys Val Lys Lys Arg Lys Gly Lys Ser
785                 790                 795

<210> 16
<211> 796

<212> PRT
<213> Neisseria meningitidis

<400> 16
Met Ser Thr Val Gln Met Asn Lys Asn Ile Lys Ser Leu Asn Leu Arg
1               5                   10                  15

Glu Lys Asp Pro Phe Leu Ser Arg Glu Lys Gln Arg Tyr Glu His Pro
            20                  25                  30

Leu Pro Ser Arg Glu Trp Ile Ile Glu Leu Leu Glu Arg Lys Gly Val
        35                  40                  45

Pro Ser Lys Ile Glu Ala Leu Val Arg Glu Leu Ser Ile Lys Glu Glu
    50                  55                  60

Glu Tyr Glu Phe Phe Glu Arg Arg Leu Lys Ala Met Ala Arg Asp Gly
65                  70                  75                  80

Gln Val Leu Ile Asn Arg Arg Gly Ala Val Cys Ala Ala Asp Lys Leu
                85                  90                  95

Asp Leu Val Lys Cys Arg Val Glu Ala His Lys Asp Gly Phe Gly Phe
            100                 105                 110

Ala Val Pro Leu Thr Pro Ala Lys Asp Gly Asp Phe Val Leu Tyr Glu
        115                 120                 125

Arg Gln Met Arg Gly Ile Met His Gly Asp Ile Val Thr Val Arg Pro
    130                 135                 140

Ala Gly Met Asp Arg Arg Gly Arg Arg Glu Gly Thr Val Leu Asp Ile
145                 150                 155                 160

Val Glu Arg Ala Gln Ser Lys Val Val Gly Arg Phe Tyr Met Asp Arg
                165                 170                 175

Gly Val Ala Ile Leu Glu Pro Glu Asp Lys Arg Leu Asn Gln Ser Ile
            180                 185                 190

Val Leu Glu Pro Asp Gly Val Ala Arg Phe Lys Pro Glu Ser Gly Gln
        195                 200                 205

Val Ile Val Gly Glu Ile Glu Val Tyr Pro Glu Gln Asn Arg Pro Ala
    210                 215                 220

Val Ala Lys Ile Ile Glu Val Leu Gly Asp Tyr Ala Asp Ser Gly Met
225                 230                 235                 240

Glu Ile Glu Ile Ala Val Arg Lys His His Leu Pro His Gln Phe Ser
                245                 250                 255

Glu Ala Cys Ala Lys Ala Ala Lys Lys Ile Pro Asp His Val Arg Lys
            260                 265                 270

Ser Asp Leu Lys Gly Arg Val Asp Leu Arg Asp Leu Pro Leu Val Thr
        275                 280                 285

Ile Asp Gly Glu Thr Ala Arg Asp Phe Asp Asp Ala Val Phe Ala Glu
    290                 295                 300

Lys Ile Gly Arg Asn Tyr Arg Leu Val Val Ala Ile Ala Asp Val Ser
305                 310                 315                 320

His Tyr Val Arg Pro Asp Asp Ala Ile Asp Thr Asp Ala Gln Glu Arg
                325                 330                 335

Ser Thr Ser Val Tyr Phe Pro Arg Arg Val Ile Pro Met Leu Pro Glu
            340                 345                 350

Asn Leu Ser Asn Gly Ile Cys Ser Leu Asn Pro His Val Glu Arg Leu
        355                 360                 365

Cys Val Val Cys Asp Met Val Ile Thr Tyr Ala Gly Asn Ile Lys Glu
    370                 375                 380

Tyr Arg Phe Tyr Pro Ala Val Met Arg Ser His Ala Arg Leu Thr Tyr
385                 390                 395                 400

Asn Gln Val Trp Lys Trp Leu Ser Gly Gly Ile Glu His Pro Phe Lys
                405                 410                 415

Thr Gln Ile Asp Thr Leu Tyr Lys Leu Phe Lys Ile Leu Gln Lys Lys
            420                 425                 430

Arg Phe Glu Arg Gly Ala Val Glu Phe Asp Ser Ile Glu Thr Gln Met
        435                 440                 445

Leu Phe Asp Asp Asn Gly Lys Ile Glu Lys Ile Val Pro Val Val Arg
    450                 455                 460

Asn Asp Ala His Lys Leu Ile Glu Glu Cys Met Leu Ala Ala Asn Val
465                 470                 475                 480

Cys Ala Ala Asp Phe Leu Leu Lys Asn Lys His Thr Ala Leu Phe Arg
                485                 490                 495

Asn His Leu Gly Pro Thr Pro Glu Lys Leu Ala Ala Leu Arg Glu Gln
            500                 505                 510

Leu Gly Leu Leu Gly Leu Gln Leu Gly Gly Gly Asp Asn Pro Ser Pro
        515                 520                 525

Lys Asp Tyr Ala Ala Leu Ala Gly Gln Phe Lys Gly Arg Pro Asp Ala
    530                 535                 540

Glu Leu Leu Gln Val Met Met Leu Arg Ser Met Gln Gln Ala Val Tyr
545                 550                 555                 560

Glu Pro His Cys Asp Gly His Phe Gly Leu Ala Tyr Glu Ala Tyr Ala
                565                 570                 575

His Phe Thr Ser Pro Ile Arg Arg Tyr Pro Asp Leu Thr Val His Arg
            580                 585                 590

Ala Ile Lys Ala Val Leu Asn Gln Gln Thr Tyr Thr Pro Lys Lys Ser
        595                 600                 605

Trp Gln Ala Leu Gly Val His Thr Ser Phe Cys Glu Arg Arg Ala Asp
    610                 615                 620

Asp Ala Ser Arg Asp Val Glu Asn Trp Leu Lys Thr Tyr Tyr Met Arg
625                 630                 635                 640

Asp Lys Val Gly Glu Val Phe Glu Gly Lys Ile Ser Gly Met Thr Ser
                645                 650                 655

Phe Gly Ile Phe Val Thr Leu Asp Gly Ile His Ile Asp Gly Leu Val
            660                 665                 670

His Ile Ser Asp Leu Gly Glu Asp Tyr Phe Asn Phe Arg Pro Glu Ile
        675                 680                 685

Met Ala Ile Glu Gly Glu Arg Ser Gly Ile Arg Phe Asn Met Gly Asp
    690                 695                 700

Arg Val Ala Val Arg Val Ala Arg Ala Asp Leu Asp Asp Gly Lys Ile
705                 710                 715                 720

Asp Phe Val Leu Ile Ala Gly Gly Ser Gly Arg Gly Arg Lys Val Lys
                725                 730                 735

Ser Ser Ala Ser Ala Lys Pro Ala Gly Thr Ala Gly Lys Gly Lys Pro
            740                 745                 750

Lys Thr Ala Ala Glu Lys Lys Thr Ala Arg Gly Gly Lys Val Arg Gly
        755                 760                 765

Arg Gly Ala Ser Ala Ala Ala Glu Ser Arg Lys Lys Ala Lys Lys Pro
    770                 775                 780

Val Pro Ile Lys Val Lys Lys Arg Lys Gly Lys Ser
785                 790                 795

<210> 17
<211> 252
<212> DNA
<213> Neisseria meningitidis

<220>
<221> CDS
<222> (1)..(252)

<400> 17
atg gag cag tcg ggc aaa ttc agt tgg tct gcg gca gct ttt tgg gac 48
Met Glu Gln Ser Gly Lys Phe Ser Trp Ser Ala Ala Ala Phe Trp Asp
1               5                   10                  15

att ccc tac ccc gcc acc agg cgg att gcc tca agt ttg tat tcg acc 96
Ile Pro Tyr Pro Ala Thr Arg Arg Ile Ala Ser Ser Leu Tyr Ser Thr
            20                  25                  30

gaa tat ttt gtc gta tgc ttt cta cgt ttg atg cca ctc tct ccg tgt 144
Glu Tyr Phe Val Val Cys Phe Leu Arg Leu Met Pro Leu Ser Pro Cys
        35                  40                  45

aat ctg tat ttt gtc acc cat ctg cgt acc aat gaa tcg gaa ata gaa 192
Asn Leu Tyr Phe Val Thr His Leu Arg Thr Asn Glu Ser Glu Ile Glu
    50                  55                  60

aga tgg tct gct gtt ccc tgc caa ata gta ttg aac gac ggc aag tcg 240
Arg Trp Ser Ala Val Pro Cys Gln Ile Val Leu Asn Asp Gly Lys Ser
65                  70                  75                  80

gaa ttc atc tga 252
Glu Phe Ile

<210> 18
<211> 83

<212> PRT
<213> Neisseria meningitidis

<400> 18
Met Glu Gln Ser Gly Lys Phe Ser Trp Ser Ala Ala Ala Phe Trp Asp
1               5                   10                  15

Ile Pro Tyr Pro Ala Thr Arg Arg Ile Ala Ser Ser Leu Tyr Ser Thr
            20                  25                  30

Glu Tyr Phe Val Val Cys Phe Leu Arg Leu Met Pro Leu Ser Pro Cys
        35                  40                  45

Asn Leu Tyr Phe Val Thr His Leu Arg Thr Asn Glu Ser Glu Ile Glu
    50                  55                  60

Arg Trp Ser Ala Val Pro Cys Gln Ile Val Leu Asn Asp Gly Lys Ser
65                  70                  75                  80

Glu Phe Ile

<210> 19
<211> 789
<212> DNA
<213> Neisseria meningitidis

<220>
<221> CDS
<222> (1)..(789)

<400> 19
atg aat gtt tat ttt ctc ggc ggc ggc aat atg gcg gct gcc gtt gcg     48
Met Asn Val Tyr Phe Leu Gly Gly Gly Asn Met Ala Ala Ala Val Ala
1               5                   10                  15

ggc gga ttg gtc aaa caa ggc ggt tac cgc atc cat ata gcc aat cgg     96
Gly Gly Leu Val Lys Gln Gly Gly Tyr Arg Ile His Ile Ala Asn Arg
            20                  25                  30

ggt gcg gaa aaa cgc gaa cgt ttg gga aaa gag ttg ggg gtc gaa act     144
Gly Ala Glu Lys Arg Glu Arg Leu Gly Lys Glu Leu Gly Val Glu Thr
        35                  40                  45

tcg gca acc ctg ccg gag ctt cat tcc gac gat gtt tta atc ctt gcc     192
Ser Ala Thr Leu Pro Glu Leu His Ser Asp Asp Val Leu Ile Leu Ala
    50                  55                  60

gtc aaa ccg cag gat atg gaa gcc gcg tgc aaa aat atc cgc acc aat 240
Val Lys Pro Gln Asp Met Glu Ala Ala Cys Lys Asn Ile Arg Thr Asn
65                  70                  75                  80

ggt gca ttg gtg ctt tct gtc gca gct gga ttg tcg gtt gat acg ctc 288
Gly Ala Leu Val Leu Ser Val Ala Ala Gly Leu Ser Val Asp Thr Leu
                85                  90                  95

agc cgt tac ctc ggg gga aca cgc cgc att gtc cgg gtt atg ccg aat 336
Ser Arg Tyr Leu Gly Gly Thr Arg Arg Ile Val Arg Val Met Pro Asn
            100                 105                 110

aca ccc gga aaa atc ggg ctg ggc gta tcc ggt atg ttt gcc gaa gcg 384
Thr Pro Gly Lys Ile Gly Leu Gly Val Ser Gly Met Phe Ala Glu Ala
        115                 120                 125

gaa gta tcg gaa aca gac cgc agg att gcc gat cga atc atg aaa tca 432
Glu Val Ser Glu Thr Asp Arg Arg Ile Ala Asp Arg Ile Met Lys Ser
    130                 135                 140

gtc ggt ttg acc gtt tgg ttg gaa gat gaa gcg caa atg cac agc att 480
Val Gly Leu Thr Val Trp Leu Glu Asp Glu Ala Gln Met His Ser Ile
145                 150                 155                 160

acc ggt atc agc ggc agc ggg ccg gca tat gtg ttt tat ctg ctg gac 528
Thr Gly Ile Ser Gly Ser Gly Pro Ala Tyr Val Phe Tyr Leu Leu Asp
                165                 170                 175

gca ttg caa aat gcc gcc atc cga caa ggg ttt gat atg gca gaa gca 576
Ala Leu Gln Asn Ala Ala Ile Arg Gln Gly Phe Asp Met Ala Glu Ala
            180                 185                 190

cgc gcg ctc agt ctg gca acg ttt aaa gga gcg gtt gcc ctt gcc gag 624
Arg Ala Leu Ser Leu Ala Thr Phe Lys Gly Ala Val Ala Leu Ala Glu
        195                 200                 205

cag acg ggt gaa gat ttc gag aag ctt caa aaa aat gta acg tca aaa 672
Gln Thr Gly Glu Asp Phe Glu Lys Leu Gln Lys Asn Val Thr Ser Lys
    210                 215                 220

ggc ggg aca acc cac gaa gcc gtg gaa gct ttc agg cgg cat cgt gtc 720
Gly Gly Thr Thr His Glu Ala Val Glu Ala Phe Arg Arg His Arg Val
225                 230                 235                 240

gcc gaa gcc ata agc gag ggc gtt tgt gcc tgt gtg cgc cgt tcg cag 768
Ala Glu Ala Ile Ser Glu Gly Val Cys Ala Cys Val Arg Arg Ser Gln
                245                 250                 255

gaa atg gaa cgg caa tat caa                                          789
Glu Met Glu Arg Gln Tyr Gln
            260

<210> 20
<211> 263
<212> PRT
<213> Neisseria meningitidis

<400> 20
Met Asn Val Tyr Phe Leu Gly Gly Gly Asn Met Ala Ala Ala Val Ala
1               5                   10                  15

Gly Gly Leu Val Lys Gln Gly Gly Tyr Arg Ile His Ile Ala Asn Arg
            20                  25                  30

Gly Ala Glu Lys Arg Glu Arg Leu Gly Lys Glu Leu Gly Val Glu Thr
        35                  40                  45

Ser Ala Thr Leu Pro Glu Leu His Ser Asp Asp Val Leu Ile Leu Ala
    50                  55                  60

Val Lys Pro Gln Asp Met Glu Ala Ala Cys Lys Asn Ile Arg Thr Asn
65                  70                  75                  80

Gly Ala Leu Val Leu Ser Val Ala Ala Gly Leu Ser Val Asp Thr Leu
                85                  90                  95

Ser Arg Tyr Leu Gly Gly Thr Arg Arg Ile Val Arg Val Met Pro Asn
            100                 105                 110

Thr Pro Gly Lys Ile Gly Leu Gly Val Ser Gly Met Phe Ala Glu Ala
        115                 120                 125

Glu Val Ser Glu Thr Asp Arg Arg Ile Ala Asp Arg Ile Met Lys Ser
    130                 135                 140

Val Gly Leu Thr Val Trp Leu Glu Asp Glu Ala Gln Met His Ser Ile
145                 150                 155                 160

Thr Gly Ile Ser Gly Ser Gly Pro Ala Tyr Val Phe Tyr Leu Leu Asp
                165                 170                 175

Ala Leu Gln Asn Ala Ala Ile Arg Gln Gly Phe Asp Met Ala Glu Ala
            180                 185                 190

Arg Ala Leu Ser Leu Ala Thr Phe Lys Gly Ala Val Ala Leu Ala Glu
        195                 200                 205

Gln Thr Gly Glu Asp Phe Glu Lys Leu Gln Lys Asn Val Thr Ser Lys
    210                 215                 220

Gly Gly Thr Thr His Glu Ala Val Glu Ala Phe Arg Arg His Arg Val
225                 230                 235                 240

Ala Glu Ala Ile Ser Glu Gly Val Cys Ala Cys Val Arg Arg Ser Gln
                245                 250                 255

Glu Met Glu Arg Gln Tyr Gln
            260

<210> 21
<211> 132
<212> DNA
<213> Neisseria meningitidis

<220>
<221> CDS
<222> (1)..(132)

<400> 21
atg ggg aaa cgg ctg atg ccg tct gaa ggg aaa ggt gcg gca ggc aat     48
Met Gly Lys Arg Leu Met Pro Ser Glu Gly Lys Gly Ala Ala Gly Asn
  1               5                  10                  15

ccg gag gcg cat aaa aaa gca gat ata ttc gga ctg cac ctc ccg aat     96
Pro Glu Ala His Lys Lys Ala Asp Ile Phe Gly Leu His Leu Pro Asn
             20                  25                  30

ata tct gcc tgc tgt ttc ctc ttt att cag cct tta                     132
Ile Ser Ala Cys Cys Phe Leu Phe Ile Gln Pro Leu
         35                  40

<210> 22
<211> 44
<212> PRT
<213> Neisseria meningitidis

<400> 22
Met Gly Lys Arg Leu Met Pro Ser Glu Gly Lys Gly Ala Ala Gly Asn
  1               5                  10                  15

Pro Glu Ala His Lys Lys Ala Asp Ile Phe Gly Leu His Leu Pro Asn
            20                  25                  30
Ile Ser Ala Cys Cys Phe Leu Phe Ile Gln Pro Leu
        35                  40

## Description

### Field of the Invention

This invention relates to bacterial genes and proteins, and their use. More particularly, it relates to genes and proteins/peptides obtained from *Neisseria meningitidis*, and their use in therapy and in screening for drugs.

### Background of the Invention

*Neisseria meningitidis* is a Gram-negative bacterial pathogen that is implicated in septic shock and bacterial meningitis. This bacterium is a leading cause of bacterial meningitis in developed countries, and causes large-scale epidemics in Africa and China. In the UK, *Neisseria meningitidis* is the leading cause of death in childhood apart from road traffic accidents. The bacterium naturally inhabits the human naso-pharynx and then gains access to the blood stream from where it causes severe septicaemia or meningitis. Although current anti-microbials are effective in eliminating the bacterium from the body, the mortalilty from menigococcal septicaemia remains substantial.

Immunisation with avirulent *N. meningitidis* microorganisms has also been proposed. Avirulence is achieved through genetic modification to disrupt the expression of genes implicated in virulence. One concern is that a mutant strain may revert to its virulent form by taking up replacement DNA. It is therefore desirable to provide means for treating or preventing conditions caused by *Neisseria meningitidis*, e.g. by immunisation.

### Summary of the Invention

The present invention is based on the discovery of genes in *Neisseria meningitidis* that are required for natural competence (uptake and incorporation of exogenous DNA).

The genes identified herein may be utilised to prepare microorganisms that are transformation-deficient. The mutant microorganisms will usually have a mutation that disrupts the expression of one or more of the genes identified herein, to provide a strain that lacks the ability to uptake DNA. These microorganisms will also have use in therapy and diagnosis.

According to a first aspect of the invention, a microorganism comprises a mutation that disrupts the expression of a gene comprising any of the nucleotide sequences identified in claim 1, or a homologue thereof with at least 40% sequence identity, for therapeutic or diagnostic use.

The microorganisms may have many therapeutic uses for treating *Neisseria* infections, including use in vaccines for prophylactic application.

According to a second aspect, a peptide is encoded by an operon including any of the nucleotide sequences identified in claim 1.

### Description of the Invention

The present invention is based on the discovery of genes encoding peptides which are responsible for the uptake of DNA. The peptides and genes of the invention are therefore useful for the preparation of therapeutic agents to treat infection. It should be understood that references to therapy also include preventative treatments, e.g. vaccination. Furthermore, while the products of the invention are intended primarily for treatment of infections in human patients, veterinary applications are also considered to be within the scope of the invention.

The present invention is described with reference to *Neisseria meningitidis.* However, all the *Neisseria* strains, and many other Gram-negative bacterial strains are likely to include related peptides or proteins having amino acid sequence identity or similarity to those identified herein. Organisms likely to contain the peptides include, but are not limited to the genera *Salmonella, Enterobacter, Klebsiella, Shigella* and *Yersinia.*

Preferably, the peptides that may be useful in the various aspects of the invention have greater than a 40% similarity with the peptides identified herein. More preferably, the peptides have greater than 60% sequence similarity. Most preferably, the peptides have greater than 80% sequence similarity, e.g. 95% similarity. With regard to the polynucleotide sequences identified herein, related polynucleotides that may be useful in the various aspects of the invention may have greater than 40% identity with the sequences identified herein. More preferably, the polynucleotide sequences have greater than 60% sequence identity. Most preferably, the polynucleotide sequences have greater than 80% sequence identity, e.g. 95% identity.

The terms "similarity" and "identity" are known in the art. The use of the term "identity" refers to a sequence comparison based on identical matches between correspondingly identical positions in the sequences being compared. The term "similarity" refers to a comparison between amino acid sequences, and takes into account not only identical amino acids in corresponding positions, but also functionally similar amino acids in corresponding positions. Thus similarity between polypeptide sequences indicates functional similarity, in addition to sequence similarity.

Levels of identity between gene sequences and levels of identity or similarity between amino acid sequences can be calculated using known methods. In relation to the present invention, publicly available computer based methods for determining identity and similarity include the BLASTP, BLASTN and FASTA (Atschul et al., J. Molec. Biol., 1990; 215:403-410), the BLASTX program available from NCBI, and the Gap program from Genetics Computer Group, Madison WI. The levels of similarity and identity provided herein, were obtained using the Gap program, with a Gap penalty of 12 and a Gap length penalty of 4 for determining the amino acid sequence comparisons, and a Gap penalty of 50 and a Gap length penalty of 3 for the polynucleotide sequence comparisons.

Having characterised a gene according to the invention, it is possible to use the gene sequence to search for related genes or peptides in other microorganisms. This may be carried out by searching in existing databases, e.g. EMBL or GeneBank.

Peptides or proteins according to the invention may be purified and isolated by methods known in the art. In particular, having identified the gene sequence, it will be possible to use recombinant techniques to express the genes in a suitable host. Active fragments and related molecules can be identified and may be useful in therapy. For example, the peptides or their active fragments may be used as antigenic determinants in a vaccine, to elicit an immune response. They may also be used in the preparation of antibodies, for passive immunisation, or diagnostic applications. Suitable antibodies include monoclonal antibodies, or fragments thereof, including single chain Fv fragments. Methods for the preparation of antibodies will be apparent to those skilled in the art.

Active fragments of the peptides are those that retain the biological function of the peptide, i.e. retain the ability to take up DNA. Typically, the fragment will be at least 30 nucleotides (10 amino acids) in size, preferably 60 nucleotides (20 amino acids) and most preferably greater than 90 nucleotides (30 amino acids) in size.

The gene's identified herein are likely to be useful both in generating vaccine strains that cannot take up exogenous DNA, and as a target for antimicrobials. Attenuated microorganisms can be produced with an attenuating mutation in a virulence gene or genes together with a mutation that disrupts a gene or genes of the invention, providing an attenuated microorganism that cannot take up extracellular DNA.

The term "virulence" is known in the art and refers to the pathogenicity of a microorganism. The use of the term "avirulent" is intended therefore to refer to microorganisms that are no longer pathogenic.

Suitable virulence genes will be apparent to the skilled person, and include auxotroph genes and those identified in Sun et al., Nature medicine, 2000; 6(11): 1269-1273.

The skilled person will be aware of methods for disrupting the expression of particular genes. Techniques that may be used include insertional inactivation or gene deletion techniques. Attenuated microorganisms according to the invention may also comprise additional mutations in other genes.

The preparation of vaccines based on attenuated microorganisms is known to those skilled in the art. Vaccine compositions can be formulated with suitable carriers or adjuvants, e.g. alum, as necessary or desired, to provide effective immunisation against infection. The preparation of vaccine formulations will be apparent to the skilled person.

Attenuated microorganisms may also be used as carrier systems for the delivery of heterologous antigens, therapeutic proteins or nucleic acids (DNA or RNA). In this embodiment, the attenuated microorganisms are used to deliver a heterologous antigen, protein or nucleic acid to a particular site in vivo. Introduction of a heterologous antigen, peptide or nucleic acid into an attenuated microorganism can be carried out by conventional techniques, including the use of recombinant constructs, e.g. vectors, which comprise polynucleotides that express the heterologous antigen or therapeutic protein, and also include suitable promoter sequences. As the microorganisms are transformation-deficient, it will be necessary to incorporate heterologous nucleic acids via an active mechanism, e.g. electroporation. This is a known technique. Alternatively, the gene that encodes the heterologous antigen or protein may be incorporated into the genome of the organism and the endogenous promoters used to control expression. A further alternative is to modify the microorganisms to disrupt the genes identified herein after first transforming with the heterologous gene.

More generally, and as is well known to those skilled in the art, a suitable amount of an active component of the invention can be selected, for therapeutic use, as can suitable carriers or excipients, and routes of administration. These factors would be chosen or determined according to known criteria such as the nature/severity of the condition to be treated, the type and/or health of the subject etc.

The various products of the invention may also be used in veterinary applications.

The following is a brief overview of the experimental procedure used to identify the genes.

Signature-tagged mutagenesis (STM) (Hensel et al., Science, 1995; 269: 400-403) was used to identify the genes in *N. meningitidis* that are essential for uptake of DNA. In STM, individual mutants are tagged with unique sequence identifiers, allowing large numbers of mutants to be analysed simultaneously.

A *Neisseria meningitidis* serogroup B Tn10 mutant bank was used to screen for mutants that exhibited a transformation defect. Pools of mutants were transformed with either plasmid or chromosomal DNA carrying an erythromycin-resistance marker. Transformation-deficient mutants were identified by plating out transformed pools of mutant bacteria onto solid media, in the presence and absence of erythromycin and performing a STM screen. Bacteria containing a modified version of the transposon Tn10 inserted within a competence gene failed to be recovered from plates containing erythromycin inoculated with a mixed population of mutants, and were, therefore likely to be defective in the uptake of exogenous DNA.

The cloned *Neisseria meningitidis* nucleotide sequences immediately following the Tn10 insertion were identified, and their translated sequences characterised. Homologues were then searched for in existing protein and DNA databases (www.sanger.ac.uk/Projects/N-meningitidis; www.tigr.org; and www.ncbi.nlm.nih.gov).

The selected mutants were tested for the ability to take up exogenous DNA and were shown to take up exogenous DNA at levels at least 5 fold below wild-type levels. As the genes are transcribed as part of an operon it is possible that the mutation is due to a polar effect on a downstream gene.

To prevent the identification of mutants that merely could not express an erythromycin resistant or capsule-minus phenotype, all putatively competence-defective mutants were also analysed for their ability to be transformed to tetracycline resistance. The results confirmed that the mutants were transformation deficient.

The genes of the invention are shown in Table 1 together with the identification of any known homologue. In addition, Table 1 shows the transformation frequency, illustrating the deficiency of DNA uptake exhibited by the mutants, compared to wild-type.

**Table 1**

| **SEQ ID NO.** | **Genes** | **Function** | **Transformation frequency** | **Accession number** |
|---|---|---|---|---|
| 1 | *rec*G | ATP-dependent DNA helicase RECG | 1/5 | NMB1788 |
| 3 | rho | Transcription termination factor RHO | 0 | NMB0617 |
| 5 | *pil*N | PBP1 | 0 | NMB1809 |
| 7 | *phl*/l | Phosphoenolpyruvate-protein phosphotransferase | 1/30 | NMB2044 |
| 9 | *cut*E | *E. coli* apolipoprotein N-acyltransferase | 1/10 | NMB0713 |
| 11 | *smf* | SMF protein (DNA processing chain A) | 0 | NMB0116 |
| 13 | | CcsA-related protein | 1/2000 | NMB0296 |
| 15 | *yac*B | *E. coli* Vacb protein | 1/10 | NMB1200 |
| 17 | | No hit in NM nucleic acid database | 1/5 | |
| 19 | *pro*C | Pyrroline-5-carboxylate reductase | 0 | NMB055 |
| 21 | | Phosphoribosylglycinamide formyltransferase | 1/40 | NMB1566 |

## Claims

1. A microorganism comprising a mutation that disrupts the expression of a gene comprising any of the nucleotide sequences identified herein as SEQ ID NOS. 1, 3, 5, 7, 9, 11, 13, 15, 17, 19 and 21, or a related molecule having at least 40% sequence identity, for therapeutic use.

2. A microorganism according to claim 1, wherein the mutation results in insertional inactivation or a gene deletion.

3. A microorganism according to claim 1 or claim 2, wherein the microorganism is *Neisseria meningitidis.*

4. A microorganism according to any preceding claim, further comprising a mutation that disrupts the expression of a virulence gene.

5. A microorganism according to any preceding claim, comprising a heterologous antigen, therapeutic peptide or nucleic acid.

6. A microorganism according to any preceding claim, wherein the sequence identity is at least 60%.

7. A microorganism according to any preceding claim, wherein the sequence identity is at least 90%.

8. A microorganism according to any of claims 1 to 5, wherein the gene normally encodes a peptide comprising any of the amino acid sequences identified herein as SEQ ID NOS. 2, 4, 6, 8, 10, 12, 14, 16, 18, 20 or 22.

9. A vaccine comprising a microorganism according to any preceding claim.

10. A peptide encoded by an operon including any of the nucleotide sequences identified herein as SEQ ID NOS. 1, 3, 5, 7, 9. 11, 13, 15, 17, 19 and 21 of *N. meningitidis,* or a related molecule having at least 40% sequence similarity or identity at the peptide or nucleotide level in a Gram-negative bacterium, or a functional fragment thereof, for therapeutic or diagnostic use.

11. A peptide according to claim 10, wherein the sequence similarity or identity is at least 60%.

12. A peptide according to claim 10 or claim 11, wherein the sequence similarity or identity is at least 90%.

13. A peptide according to claim 10, comprising the amino acid sequence identified herein as SEQ ID NOS. 2, 4, 6, 8, 10, 12, 14, 16, 18, 20 and 22.

14. A polynucleotide encoding a peptide according to any of claims 10 to 14, or its complement, for therapeutic or diagnostic use.

15. An antibody raised against a peptide according to any of claims 10 to 13.

16. Use of a product according to any of claims 1 to 14, for the manufacture of a medicament for use in the treatment or prevention of a condition associated with infection by *Neisseria* or Gram-negative bacteria.

17. Use according to claim 16, wherein the condition is meningitis.

18. Use according to claim 16 or claim 17, for veterinary treatment.
